# EUROPEAN PATENT SPECIFICATION

(11) **EP 0 394 538 B1**
(45) Date of publication and mention of the grant of the patent: **16.10.1996**
(21) Application number: 89107780.2
(22) Date of filing: 28.04.1989
(51) Int. Cl.: C12N 1/19, C12P 21/02, C12N 15/81

(54) **A yeast cell of the genus schwanniomyces**
Hefezellen der Gattung-Schwanniomyces
Cellules de levure du genre schwanniomyces

(43) Date of publication of application: 31.10.1990
(73) Proprietor: RHEIN BIOTECH GESELLSCHAFT FUR NEUE BIOTECHNOLOGISCHE PROZESSE UND PRODUKTE MBH, 40595 Düsseldorf (DE)
(72) Inventor: Hollenberg, Cornelius P., Prof. Dr., D-4000 Düsseldorf (DE); Strasser, Alexander, Dr., D-4000 Düsseldorf (DE)
(74) Representative: Grünecker, Kinkeldey, Stockmair & Schwanhäusser Anwaltssozietät

(56) References cited:
- EP-A- 0 256 421
- EP-A- 0 257 115
- EP-A- 0 260 404
- SCIENCE, vol. 229, 20th September 1985, pages 1219-1224, Washington, DC, US; R. A. SMITH et al.: "Heterologous protein secretion from yeast"
- CRC - CRITICAL REVIEWS IN BIOTECHNOLOGY, vol. 5, no. 2, 1987, pages 159-176, Boca Raton, Florida, US; W.M. INGLEDEW.

## Description

The present invention relates to yeast cells of the genus Schwanniomyces and a process for the production of polypeptides.

For expression of eukaryotic proteins of biotechnogical relevance there exists a variety of host systems, which are selected in compliance with the specific requirements. The most frequently used host organism is Escherichia coli, being the best known representative of prokaryotic host organisms; Saccharomyces cerevisiae and animal tissue cultures are used as eukaryotic host cells. For certain purposes, e.g. where expression of a glycoprotein is desired, expression in E.coli or other prokaryotic organisms is not appropriate, since same lack any glycosylation capability. On the other hand, the mass production of protein in tissue cultures still is cumbersome and expensive; therefore, the organism of choice in many instances is yeast of the well characterized genus Saccharomyces. S. cerevisiae is able to secrete efficiently small proteins, e.g. polypeptides like MG1 (Kurjan and Herskowitz, 1982), β-endorphin or α-interferon (Bitter et al., 1984), however proteins of higher molecular weight are captured in the periplasmic space (Emr et al, 1981) or the cytoplasma, thus causing decreased growth rates or even death of the cell.

Therefore it is an object of the present invention to provide an improved eukaryotic expression system, which is optionally capable of secreting proteins encoded by a foreign DNA into the culture medium.

This object has been solved by a yeast cell of the genus Schwanniomyces, wherein said yeast cell contains at least one expression cassette comprising :
a) a first DNA sequence serving as a regulon,
b) optionally a second DNA sequence coding for a signal peptide
c) a third DNA sequence coding for a foreign protein, and
d) optionally a fourth DNA sequence serving as a terminator, wherein the first, second and fourth DNA sequence are derived from yeast.

The invention, which comprises further subjects, is now described in a more detailed manner by the following description, examples and figures.

### BRIEF DESCRIPTION OF THE FIGURES

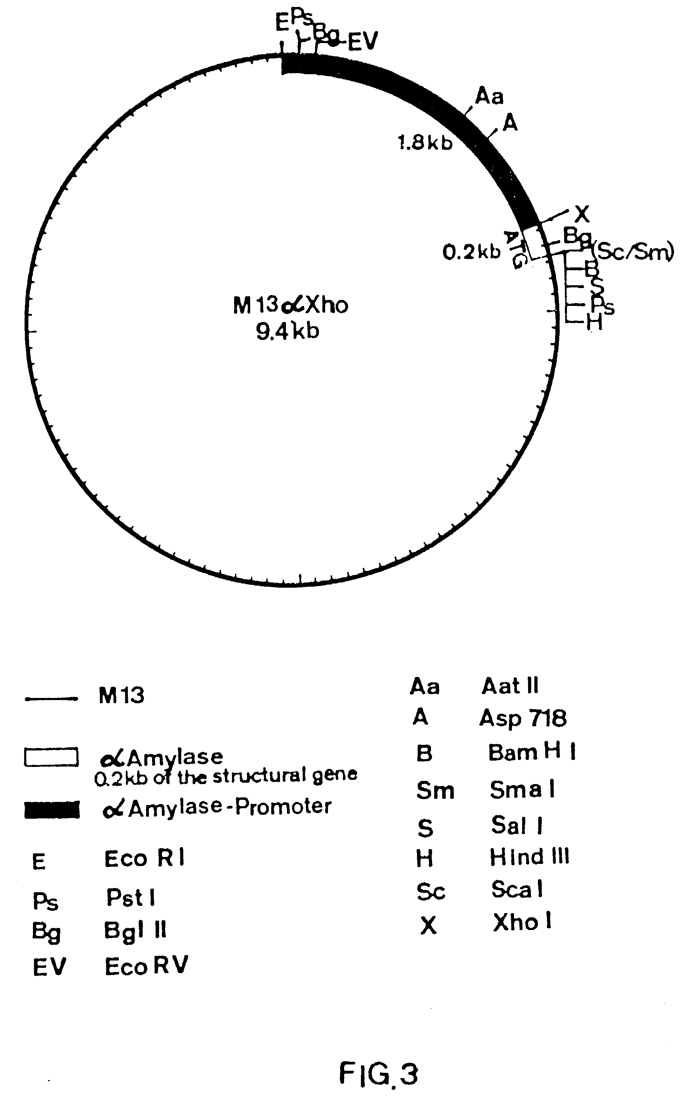

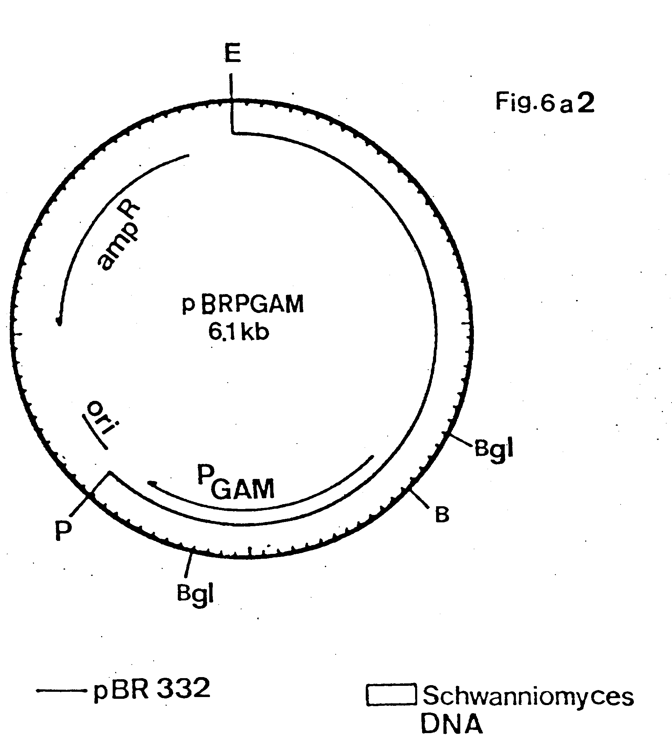

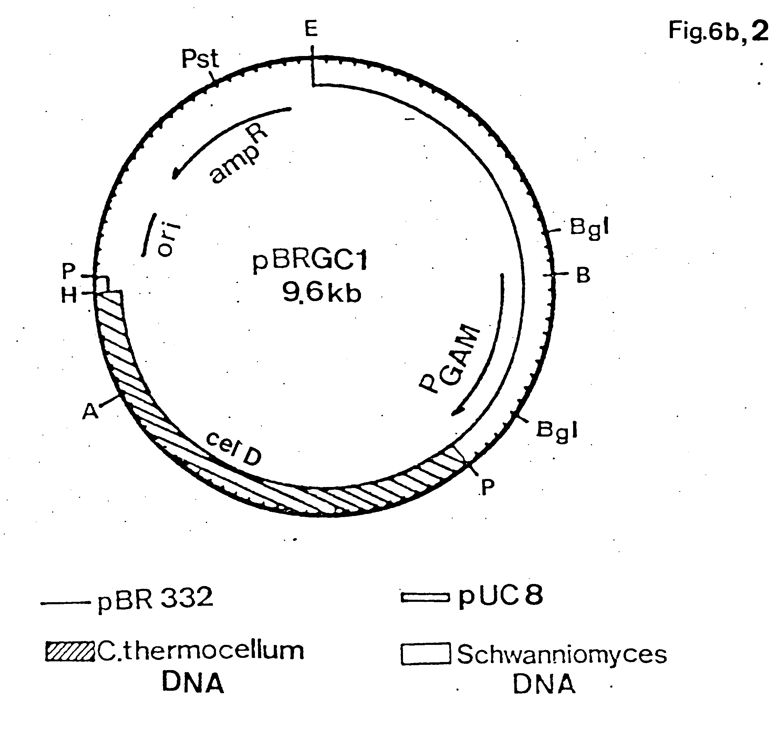

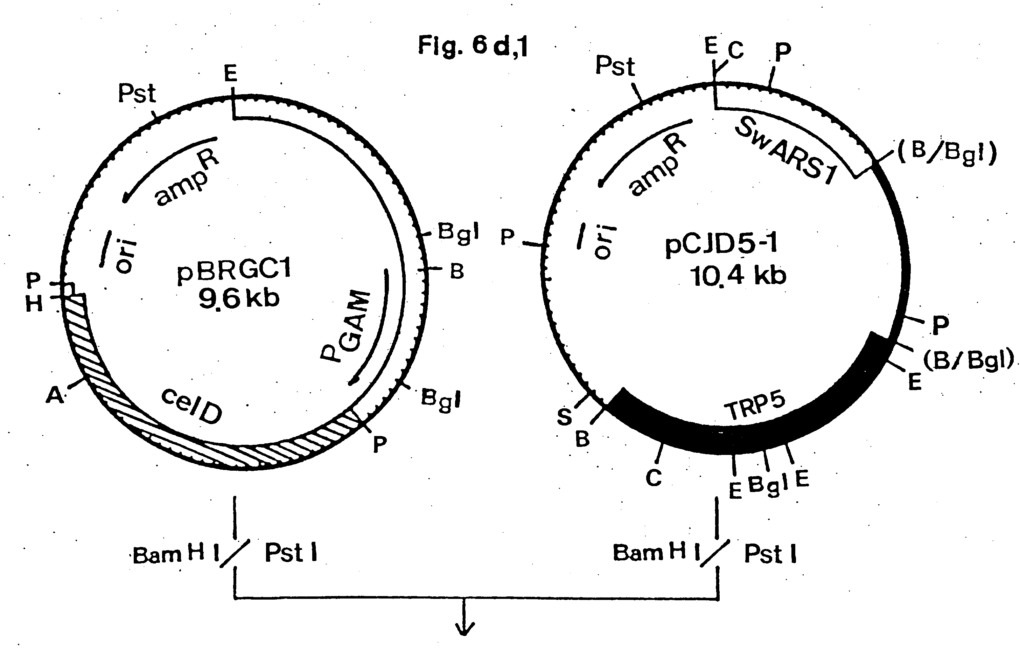

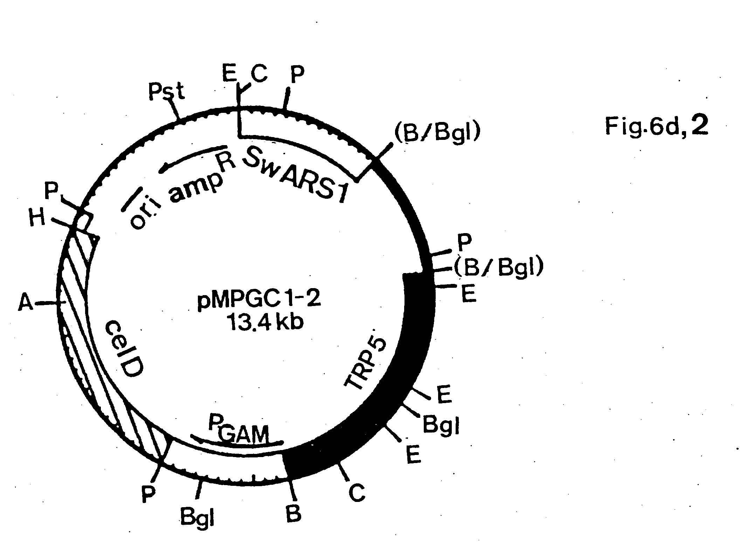

Fig.1
   A: Restriction map of the AMY1 gene encoding α-amylase and its 5' and 3' noncoding region
   B: Nucleotide sequence of the AMY1 gene and its flanking region. In the 5′ region the transcription initation site is indicated by a dashed line above the sequence. Within the structural gene the presumptive signal peptidase cleavage sites are indicated by star-symbols. The two potentiaL glycosylation sites, the cystein residues presumably forming four disulfide bonds (concluded by homology to the A. oryzae enzyme) are underlined. Within the 3' end region the transcription termination signal TAG...TATGT...TTT is indicated by a solid line.
Fig.2
   A: Restriction map of the GAM1 gene encoding glucoamylase and its 5' and 3' noncoding region
   B: Nucleotide sequence of the GAM1 gene and its flanking region. In the 5' region the transcription initiation site is indicated by a dashed line. Within the structural gene the presumptive signal peptides cleavage sites are indicated by star-symbols.
Fig.3
   M13 XhoI. The EcoRI-ScaI fragment, containing a 1.8 kb promoter fragment and approx. 0.4 kb structural gene fragment of the α-amylase gene, was subcloned into M13mp8 previously digested with EcoRI and SmaI.
   By site directed mutagenesis a XhoI-site was inserted just in front of the ATG codon: 
**   *
5'..TAAAATAAAAGCTCGAGATGAGATTT...3'.
 Positions where bases have been exchanged by site directed mutagenesis, are indicated by asterisks.
Fig.4
   pMaGAM-B/S. The BglII fragment containing the glucoamylase gene and its 5′ and 3′ flanking region was subcloned into the BamHI site of vector pMAC5-8 (Kramer et al., 1984). By site directed mutagenesis using the gapped duplex DNA methode (Kramer et al., 1984) a BamHI site and a SalI site were inserted just in front of the translation initiation codon ATG: 
* ****** **
5'.. CTCATGACTGTGTCGACGGATCCAAGATGATTTT....3'.
 Positions affected by the site specific mutagenesis are indicated by asterisks.
Fig. 5
   YRp7αGAM. The GAM gene was isolated from pMaGAM-B/S as a SalI fragment and ligated into the Xhoi site of M13αXho (Fig.3). The BglII fragment harbouring the α-amylase promoter GAM gene fusion was ligated into the single BamHI site of the S. cerevisiae vector YRp7 resulting in plasmid YRp7αGAM.
Fig.6
   Construction of S. occidentalis vectors for expression of foreign genes using the promoter and the signal sequence of the glucoamylase gene.
   a: Insertion of the 4.0 kb EcoRI-PvuII fragment from pBRSwARSGAM, containing the complete GAM promoter and the first 208 bp of the GAM encoding sequence into pRR322, cleaved with EcoRI/PvuII, resulting in vector pBRGAM.
   b: Insertion of the 3.4 kb PvuII fragment from plasmid pCT603, containing the structural gene coding for cellulase from base pair position +112, into the PvuII site of plasmid pBRGAM, resulting in vector pBRGC1.
   c: Insertion of the 5.5 kb BglII-PstI fragment from plasmid pBRGC1 into BamHI/PstI cut pCJD5-1. The resulting plasmid, pMPGC1-1 contains 321 bp of the GAM 5' non coding region and the first 208 bp of the GAM coding region fused to position +112 of the celD gene.
   d: Insertion of the 6.5 kb BamHI-PstI fragment from plasmid pBRGC1 into BamHI/PstI cut pCJD5-1. The resulting plasmid, pMPGC1-2 contains 1.3 kb of the GAM 5' non coding region and the first 208 bp of the GAM coding region fused to position +112 of the celD.
Fig. 7
   Promoter studies using the qualitative endoglucanase D activity assay (congo red staining) with different transformants: NGA58: pMPAGC, NGA58: pMPPGC, NGA58: pMPGC1-1, NGA58: pMPGC1-2
   a: Growth on 2% maltose
   b: Growth on 4% glucose
Fig.8
   Construction of a S. occidentalis vector for expression of foreign genes under control of the ADH1 promoter.
   a: Insertion of the 1.45 kb BamHI-SalI ADH1 promoter fragment of plasmid pFM2-1 into pCJD5-1, previously cut with BamHI/SalI resulting in plasmid pMPADH1.
   b: Insertion of the 3.2 kb SalI fragment of pJDcg-15 into the SalI site of pMPADH1, resulting in pMPAG.
   c: Exchange of the GAM promoter in plasmid pMPGC1-2 (Fig 6d) for the ADH1 promoter from plasmid pMPAG by in vivo recombination, resulting in plasmid pMPAGC.
Fig. 9
   Construction of a S. occidentalis vector for the expression of foreign genes under control of the PDC promoter.
   a: Insertion of the 6.0 kb SphI-AvaI fragment from plasmid pJDcg-15, containing the PDC promoter fused to the GAM gene into YRpJD2 previously cleaved with SphI-AvaI, resulting in plasmid pMPPG.
   b: Exchange of the GAM promoter in plasmid pMPGC1-2 (Fig. 6d) for the PDC promoter from plasmid pMPPG by in vivo recombination, resulting in plasmid pMPPGC.
Fig. 10
   Construction of an autonomous replicating vector (containing SwARS2) for transformation of S. occidentalis.
   The 3.2 kb TRP5 BamHI fragment from plasmid YRpJD2 was ligated with the 5.5 kb BamHI-BglII fragment of plasmid pBRSwARSGAM (Fig 6a), resulting in pMPTS2-A and pMPTS2-B.

### ABBREVIATIONS USED THROUGHOUT THE APPLICATION

Restriction endonucleases :
- Aa: -AatII
- A: - Asp718
- B: - BamHI
- Bg: - BglII
- C: - ClaI
- E: - EcoRI
- EV: - EcoRV
- H: - HindIII
- P: - PvuII
- Ps: - PstI
- S: - SalI
- Sc: - ScaI
- Sm: - SmaI
- Sp: - SphI
- X: - XhoI

Throughout this application various publications are referenced by mention of the first author and the year of publication within parentheses. Full citation of the references may be found at the end of the Specification as an annex, listed according to their alphabetical order immediately following the specification. The disclosures of these publications in their entireties are hereby incorporated by reference into this application in order to more fully describe the state of the art as known to those skilled therein as of the date of the invention described and claimed herein.

The yeast cell according to the present invention is characterized by containing an expression cassette which in turn is composed of three to four different components, being arranged in operable linkage in order to ensure efficient expression of a desired polypeptide, optionally followed by secretion of same. These components are defined to be the following :
a) A first DNA sequence serving as a regulon. The term "a regulon" as used throughout the application comprises any cis-acting DNA sequence involved in the regulation of expression of a given structural gene. The term thus embraces sequences preceeding a given coding sequence, for example, a promoter and sequences which are recognised by transcription factors or other proteins involved in transcription regulation. The "first DNA sequence" is thus not limited to sequences corresponding to known promoters. Also included are DNA sequences corresponding to natural occuring regulons, which have undergone insertion, deletion or substitution events, but still retain their activity as regulon. Further the term "regulon" includes DNA sequences involved in regulation of transcription, which have been synthesised chemically or obtained by methods of gene technology.
b) An optional second DNA sequence coding for a signal peptide. The meaning of this second DNA sequence includes various DNA sequences, directing the secretion of any polypeptide containing the thus encoded peptide at its N-terminal end.
c) A third DNA sequence coding for a foreign protein. This coding sequence coding for the signal peptide in order to retain a proper reading frame for the third DNA sequence. The fusion thus requires application of methods or recombination DNA technology known to every person skilled in the art. For methods enabling to design proper fusions it is in general referred to Maniatis et al. (1982), wherein appropriate methods, e.g. various ways to treat restriction fragment ends, cloning of linker molecules and similar procedures are described in great detail. The foreign protein coded for by the third DNA sequences may be any protein which to express there exists some need. Since the secretion system is capable of secretion not only of small molecules, but also of large proteins, expression of any desired protein should be possible. For examples it is referred to the discussion below.
d) Optionally a fourth DNA sequence serving as a terminator. As the terminator any sequence may be used, which efficiently terminates transcription in the respective host organism. Terminators may be for example sequences prone to formation of hairpin structures and/or polyadenylation sites. In a preferred embodiment the terminator is derived from the same gene as the second DNA sequence, i.e. the regulon, is derived from.

The four components are combined with each other using known techniques. Optionally the proper fusion will be verified for example by sequencing the boundaries.

The yeast cell according to the present invention contains at least one expression cassette comprising some or all of the above mentioned DNA sequences. It provides a convenient tool for the industrial expression of any desired protein.

In a preferred embodiment said first and/or second and/or fourth DNA sequence are derived from a gene coding for an amylolytic enzyme, for example α-amylase or glucoamylase, which enzymes in most organisms are induced upon contact with starch. The DNA sequences may be derived from a yeast of a member of one of the genera Debariomyces, Saccharomyces, Lipomyces, Pichia, or Saccharomyces, but yeast of the genus Schwanniomyces are preferred.

Yeast species of the genus Schwanniomyces have an efficient expression and secretion apparatus, enabling them to grow on starch as a sole carbon source. Starch is hydrolysed to glucose by two amylolytic enzymes, secreted into the supernatant, namely α-amylase (α- 1,4-glucan 4-glucanohydrolase E.C.3.2.1.1) and glucoamylase (syn. amyloglucosidase) (α-1,4-glucanglucohydrolase, E.C.3.2.1.3.; with debranching activity E.C.3.2.1.9.). The amylolytic enzymes of Schwanniomyces occidentalis, formerly also called Schwanniomyces castellii and Schwanniomyces alluvius (Price et al., 1978), are well documented (Oteng-Gyang et al., 1981; Sills et al., 1982, 1984a, 1984b; Wilson et al., 1982).

The genes for α-amylase and glucoamylase from a yeast of the genus Schwanniomyces have been disclosed by the present inventors in EP-A-0260404. However, at that time the DNA or peptide sequences directing secretion have not been identified. Furthermore their use for the establishment of an efficient expression and secretion system in yeast was not known then.

It is preferred, to use as a first DNA sequence part or all of a 1.8 kb BglII--XhoI fragment which preceeds the structural gene coding for the Schwanniomyces α-amylase, or part or all of a 1.3 kb BamHI-PvuII fragment preceeding the structural gene coding for the Schwanniomyces occidentalis glucoamylase. Further if only smaller fragments are to be used, it is preferred, to use a DNA sequence comprising part or all of the DNA sequence corresponding to bases -1 to -540 of the region preceeding the structural gene coding for α-amylase or part or all of the of DNA sequence corresponding to bases -1 to -320 of the region preceding the structural gene coding for glucoamylase. The respective DNA fragments are fully active as promoters, inducible by starch, dextrin, maltose.

It is obvious, that replacement of bases or base pairs, not involved in the transcriptional regulation, still are within the spirits of the present invention. Thus natural mutations or synthetic equivalents also are embraced by the present invention.

In yeast cells of the genus Schwanniomyces it is also possible to use promoters derived from yeast genes obtained from many other yeast genera. Preferred are regulons involved in the expression of ADH1, ADH2, PDC1, GAL1/10, PGK and GAPDH, or LAC4 which are obtained from Saccharomyces cerevisiae, Kluyveromyces Lactis or Schwanniomyces occidentalis.

Furthermore, it is possible to use viral regulons and/or terminators, for example regulons and terminators obtained from E.coli T-phages. The phages possess extraordinarily strong functional sequences for performance of their lytic functions. In a preferred embodiment sequences of phage T7 are used, which are regulated by expression of T7 RNA polymerase.

In order to increase the over all expression level and furthermore, in order to facilitate purification of produced polypeptides, it is most desirable, that the polypeptides are secreted into the culture medium. The present invention provides signal sequences having the invaluable advantage of being derived from genes of amylolytic and thus inducible enzymes, which upon induction have to be secreted very efficiently. A further advantage is, that induction of amylolytic enzymes does not require complicated media, lacking for example a specific amino acid or phosphate, but only require the culture to be transferred to a starch containing medium. Signal sequences, which are preferably used for secretion of large proteins include part or all of at least one of the following peptides:

a) MetArgPheSerThrGluGlyPheThrSerLysValValAlaAlaIle
LeuAlaPheSerArgLeuValSerAla;

b) MetArgPheSerThrGluGlyPheThrSerLysValValAlaAlaIle
LeuAlaPheSerArgLeuValSerAlaGlnProIleIlePheAspMetArg;

c) MetIlePheLeuLysLeuIleLysSerIleValIleGlyLeuGlyLeu
ValSerAla;

d) MetIlePheLeuLysLeuIleLysSerIleValIleGlyLeuGlyLeu
ValSerAlaIleGlnAlaAlaProAla;

e) MetIlePheLeuLysLeuIleLysSerIleValILeGlyLeuGlyLeu
ValSerAlaIleGlnAlaAlaProAlaSerSerIleGlySerSerAlaSerAla

In a further embodiment the above mentioned peptides are coded for by DNA sequences, which correspond to all or part of the following DNA sequences:

a) ATGAGATTTTCAACTGAAGGATTTACAAGTAAAGTTGTTGCAGCAATTTT
AGCATTCTCAAGATTGGTATCTGCT;

b) ATGAGATTTTCAACTGAAGGATTTACAAGTAAAGTTGTTGCAGCAATTT
TAGCATTCTCAAGATTGGTATCTGCTCAACCGATTATTTTTGACATGAGA;

c) ATGATTTTTCTGAAGCTGATTAAAAGTATAGTAATTGGTTTGGGATTA
GTTAGTGCT;

d) ATGATTTTTCTGAAGCTGATTAAAAGTATAGTAATTGGTTTGGGATTA
GTTAGTGCTATCCAAGCAGCCCCTGCC;

e) ATGATTTTCTGAAGCTGATTAAAAGTATAGTAATTGGTTTGGGATTA
GTTAGTGCTATCCAAGCAGCCCCTGCCTCTTCGATTGGATCTAGTGCTTCA
GCA.

It will be understood, that the replacement of amino acids by residues having comparable properties and use of alternative codons in compliance with the possibilities provided by the genetic code will not have any impact on the yeast cell according to the present invention.

The polypeptide to be expressed in a yeast cell according to the present invention is coded for by a third DNA sequence. Said third DNA sequence may comprise natural or synthetic DNA, even DNA containing intervening sequences. Examples for proteins being expressed by the yeast cell according to the present invention comprise proteins of enormous importance for scientific or medical purposes, for example cellulase, interleukine, interferon, insulin like growth factor, lymphokine, human growth factor, nerve growth factor, aprotinin, insulin, hirudin, hormones, blood clotting factors, hepatitis B surface or core antigens, viral or bacterial vaccines or human granulocyte/macrophage colony stimulating factor.

The yeast cell according to the present invention may optionally comprise a terminator as component of said expression cassette. Preferred terminators are derived from genes of one of the following genera :

Saccharomyces, Pichia, Hansenula, but preferably Schwanniomyce. Examples of preferred terminators are those derived from Schwanniomyces α-amylase gene or Schwanniomyces glucoamylase gene, part or all of which my be used. Such terminators are comprised by nucleotides 1537 to 1740 of the Schwanniomyces α-amylase gene or nucleotides 2875 to 3320 of the Schwanniomyces glucoamylase gene.

It is obvious, that in case of any of the DNA sequences mentioned modifications, which do not impair the biological function of the respective DNA fragment still are covered by the present application. There are numerous possibilities to change the DNA sequences mentioned above slightly without impairing the biological function of said fragment gene.

The yeast cell according to the present invention, carrying an expression cassette composed of the above discussed components, may be carried by a circular or linear vector, which optionally comprises one or more selective marker genes. The present inventors demonstrated, that a lot of marker genes so far used for transformation of Saccharomyces, for example TRP5, LEU2, ADE1, ADE2, HIS3, HIS4, URA3, LYS2, which may be obtained from Saccharomyces, Hansenula, Pichia or Schwanniomyces, are functional in Schwanniomyces occidentalis. Furthermore it is also possible to use the amylolytic properties of α-amylase or glucoamylase in order to select transformed organisms. The corresponding genes have been isolated and characterized. AMY1 and GAM1 are preferably obtained from Schwanniomyces.

The yeast cell according to the present invention may contain the expression cassette as an insert into one of the endogenous yeast chromosomes. Furthermore, there is however the possibility, that a DNA sequence capable of controlling autonomous replication and even also stable maintenance of the vector in the host organism, is introduced into the yeast cell. Said DNA sequences are the so called ARS sequences, capable of controlling autonomous replication and stable maintenance in the respective host organism. Said DNA sequences are preferably derived from Saccharomyces, Pichia Hansenula or Kluyveromyces, most preferred from Schwanniomyces.

In order to ensure effective replication in the prospective host organism, it is preferred to use an ARS, derived from the respective host organism.

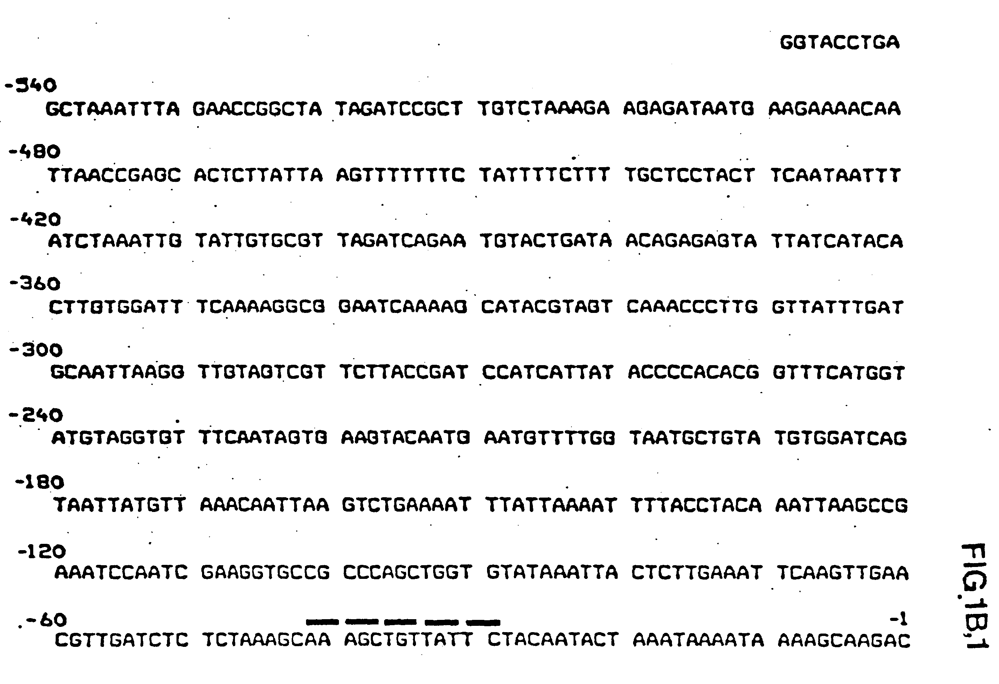

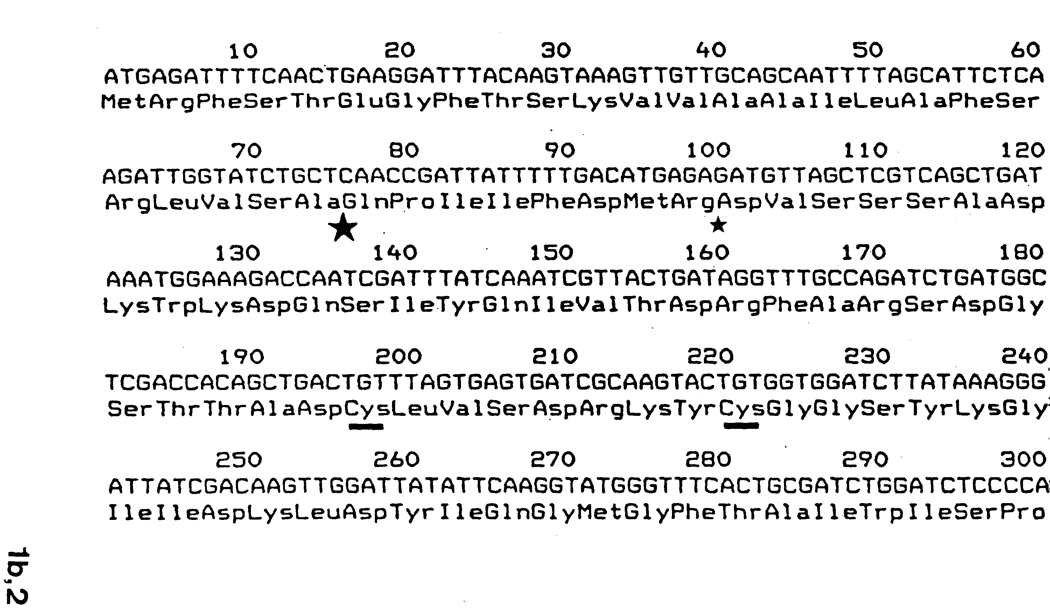

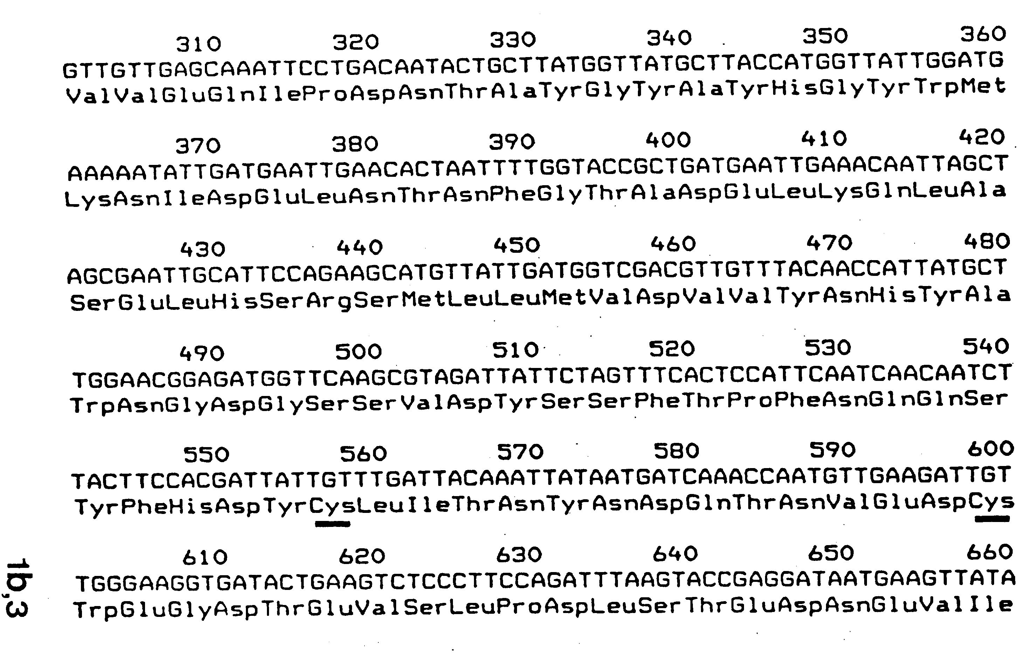

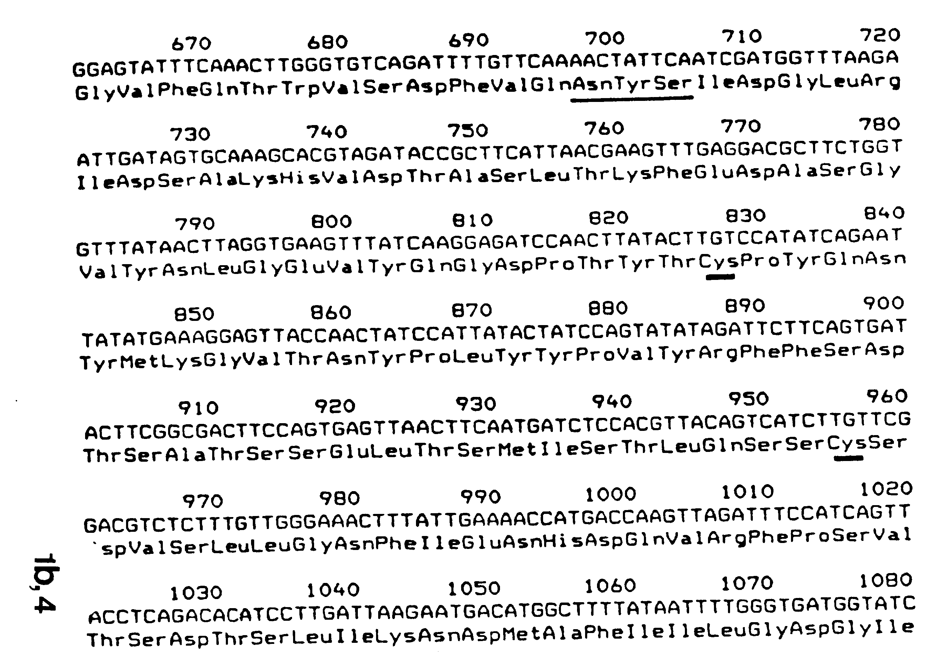

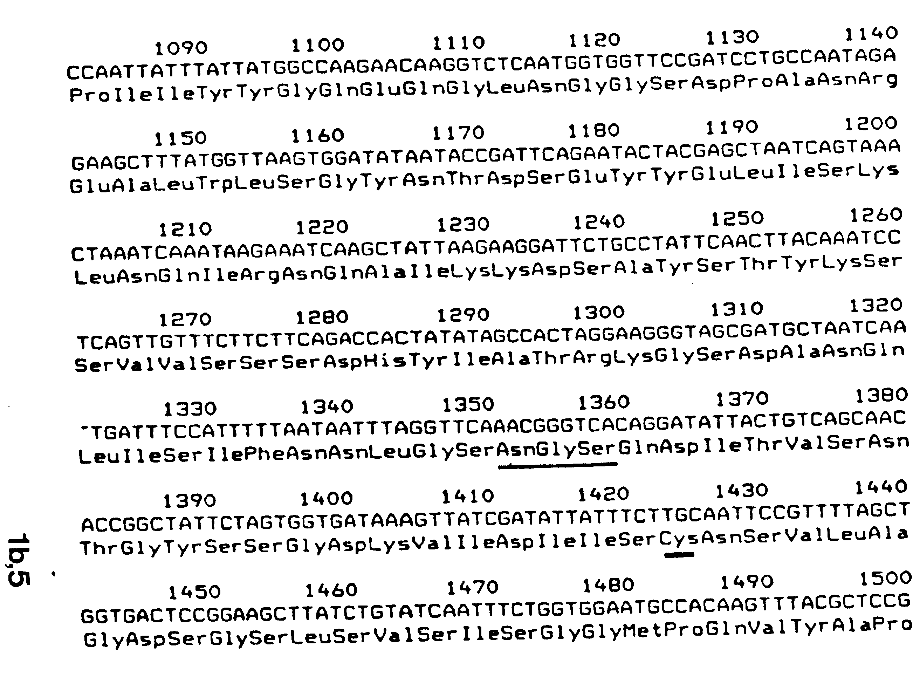

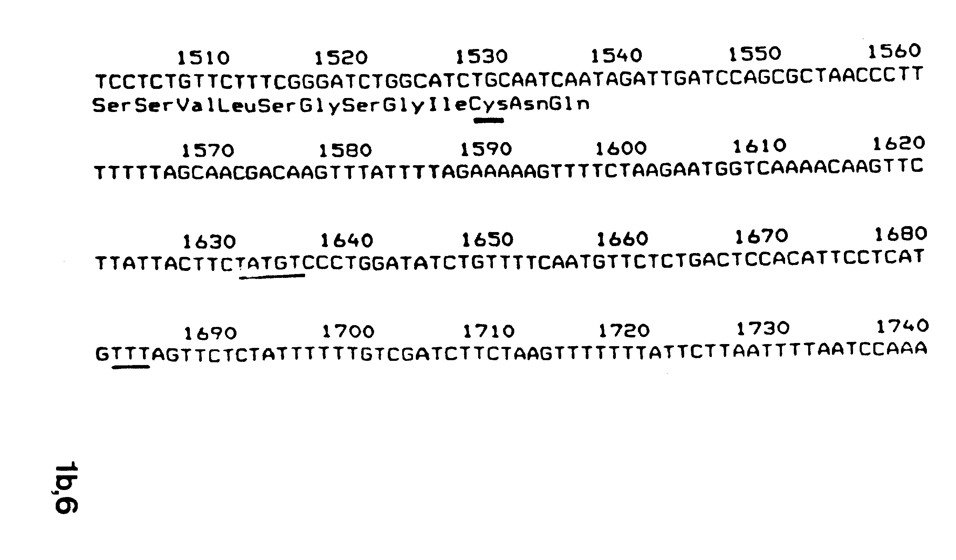

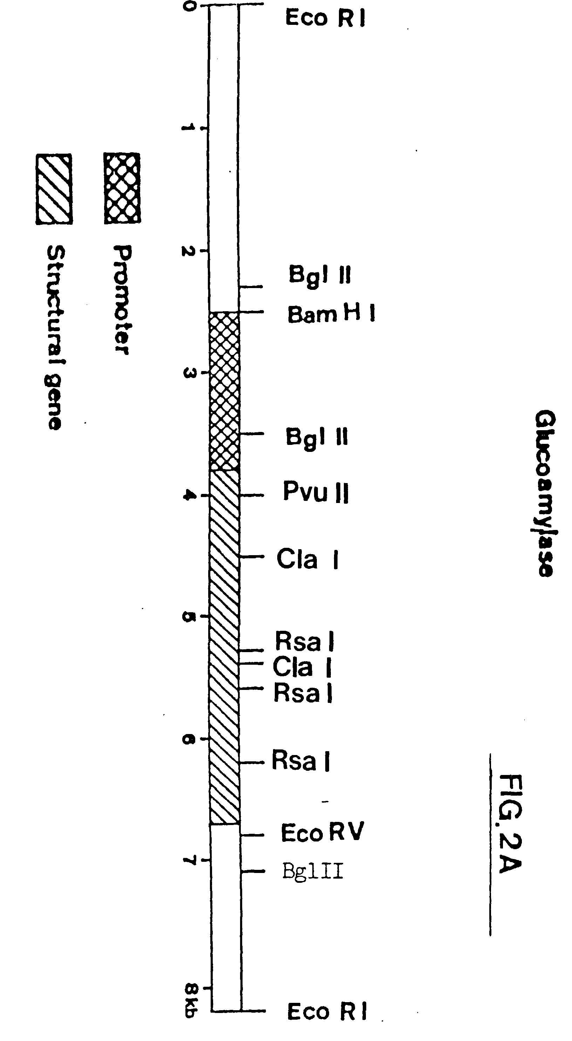

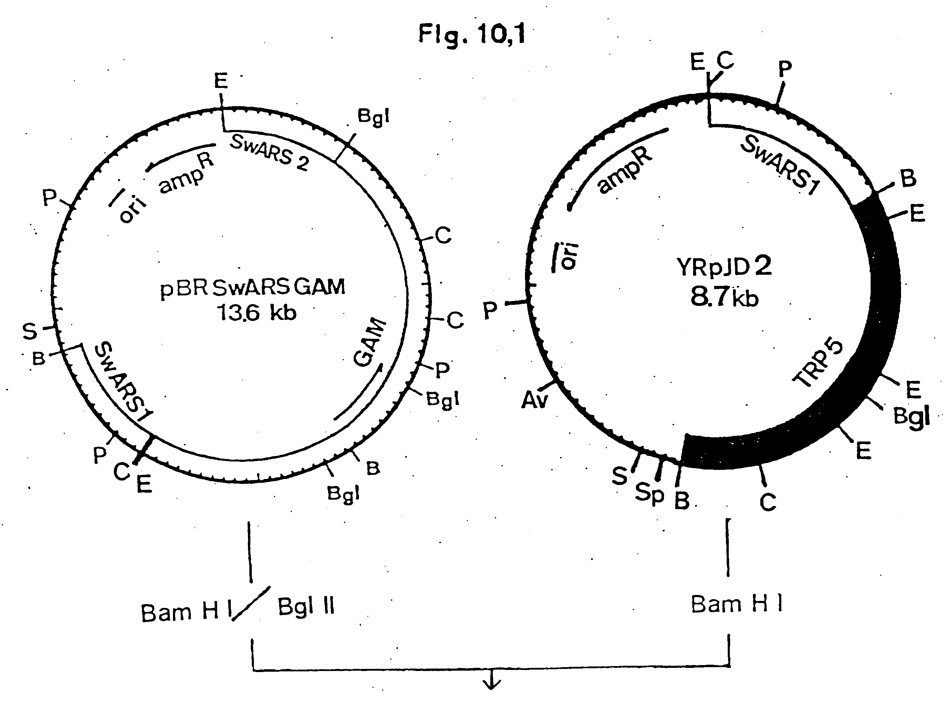

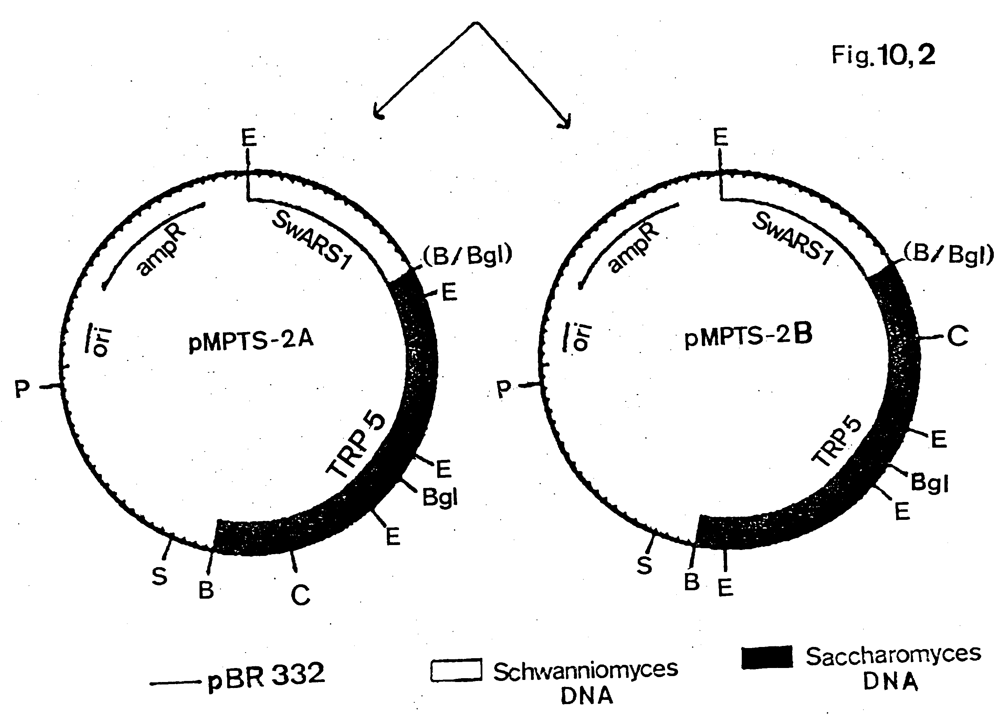

The present invention provides one new SwARS sequence. The DNA sequence SwARS2 is capable of controlling autonomous replication and stable maintenance of the vector in a Schwanniomyces yeast cell. The detailed sequence of SwARS has not yet been determined. SwARS2 is localised in the Schwanniomyces genome in the 3′ region of the glucoamylase gene, as shown for example in Fig. 10. SwARS2 is easy to identify and efficiently allows autonomous replication of any plasmid, exhibiting this sequence. The so far known Schwanniomyces autonomously replicating sequences, SwARS1 and SwARS2 are comprised on a ClaI-BamHI fragment (SwARS1) adjacent to the EcoRI-fragment shown in Fig 2a (see Fig. 10) and/or an EcoRI-BglII fragment contained in the EcoRI fragment shown in Fig 2a (SwARS2).

For the SwARS sequences it is also not necessary, in any case to use the sequence as it occurs naturally. DNA sequences, that have been obtained by any kind of modifying treatment, for example by deletion, insertion or substitution of one or more nucleotides or base pairs, are still within the scope of the present invention, provided the modifications retain or improve the biological function of the yeast cell according to the present invention.

In a preferred embodiment the yeast cell according to the present invention comprises a DNA sequence, which is homologous to genomic Schwanniomyces DNA. Provision of structures of homologous DNA enable the DNA fragment flanked by such homologous sequences to be inserted by homologous recombination. It is thus preferred to have homologous sequences, which do not represent any essential gene of the Schwanniomyces genomic DNA, but rather any gene, whose expression product may be supplemented by additives to the respective medium or were lack of the respective expression product does not have any serious impact on the transformed yeast cell.

If no SwARS sequence or any equivalent sequence, for example originating from any other yeast species, is available, the expression cassette can be used without an autonomously replicating agent. In this case the expression cassette tends to integrate into the genome, for example via homologous recombination. Preferably there are several copies inserted into one or more chromosomes.

In this case it is preferred, to have multiple copies of the expression cassette inserted, in order to ensure simultaneous transcription and translation of several copies.

When the yeast cell according to the present invention is derived from a yeast of the genus Schwanniomyces the polypeptide products observed following expression of the respective foreign gene in said yeast cells exhibit glycosylation patterns, which closely resemble or even match those of the desired eucaryotic protein. Yeast cells of the genus Schwanniomyces are thus preferred for expression of eucaryotic proteins.

The above discussed yeast cell according to the present invention can be used in a process for the production of a polypeptide, wherein a yeast host organism is cultivated under suitable conditions and the polypeptide is recovered in a manner known per se. The conditions of cultivation depend on the respective host organism used. Examples for cultivation conditions for yeast of the genus Schwanniomyces are provided in the examples below. The polypeptide, produced by the yeast according to the present invention, is recovered by conventional purification methods, which depend on the properties of the expressed protein, the availability of specific absorbing agents and so on.

In a preferred embodiment yeast cells of the species Schwanniomyces occidentalis are used as a host organism and are cultivated on starch,dextrin, maltose and/or plant biomass as the respective carbon source. As commonly known, these carbon sources are available at low costs.

Furthermore, there is a possibility to use cells of the genus Schwanniomyces for the production of single cell protein. The single cell protein may then be subjected to any further use as it is required.

In a preferred embodiment the polypeptide produced in a process according to the present invention is an amylolytic enzyme, for example an α-amylase or glucoamylase. These enzymes are commercially used to debranch and degrade starch, which thereupon may be further processed, for example in brewery or baking processes. There is a high need for the provision of starch degrading enzymes.

In a further embodiment of the process according to the present invention production of a polypeptide may be achieved by providing expression cassettes as discussed above containing a yeast α-amylase or glucoamylase signal sequence and inserting the respective expression cassette into a yeast of one of the genera Saccharomyces, Kluyveromyces, Hansenula, Pichia or Schizosaccharomyces. Production and secretion of foreign proteins in the respective yeast host organism is obtained by applying the appropriate inductive measure, whereupon the produced protein is secreted due to the presence of the yeast α-amylase or glucoamylase signal sequence. Since it is known from experiments performed by the present inventor, that these signal sequences are recognised in virtually any yeast genus, the α-amylase or glucoamylase signal sequences may be applied in many different yeast with success. Also in this case, it is preferred, to use the α-amylase or glucoamylase signal sequences derived from the respective genes of Schwanniomyces occidentalis.

In summary the yeast cells according to the present invention are well suited for transformation and expression of any desired foreign gene . As already outlined above, expression of procaryotic as well as eucaryotic genes is possible. However, for expression of eucaryotic proteins yeast cells of the genus Schwanniomyces containing some or all of the DNA sequences mentioned above are the most preferred yeast cells.

### CHARACTERISATION OF THE α-AMYLASE AND GLUCOAMYLASE GENE EXPRESSION IN DIFFERENT YEAST GENERA :

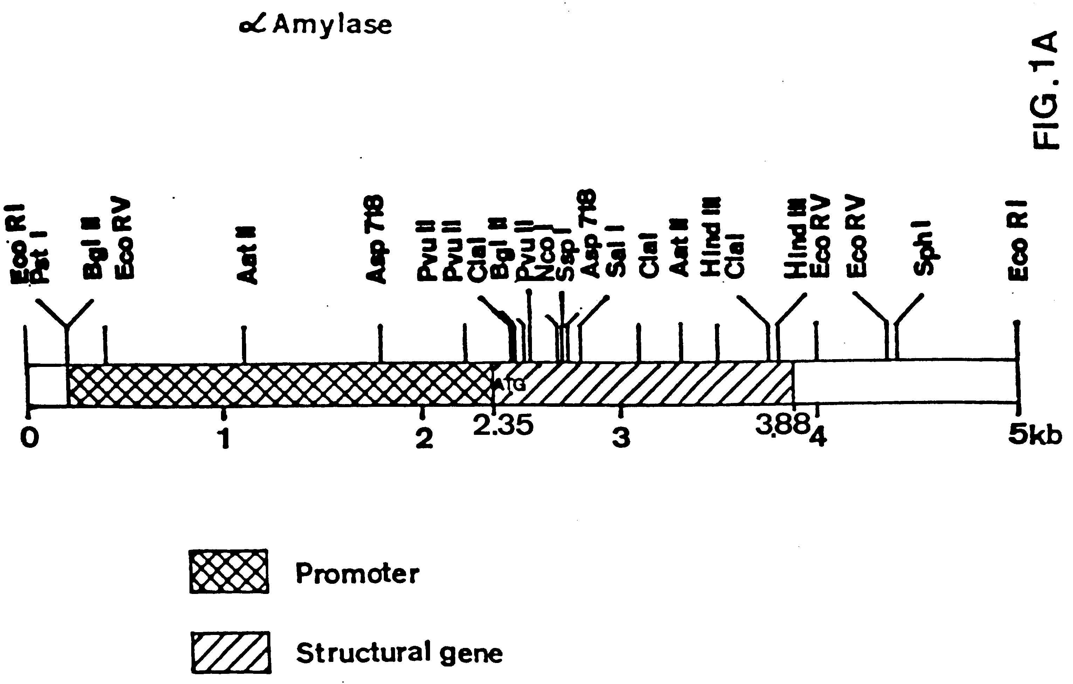

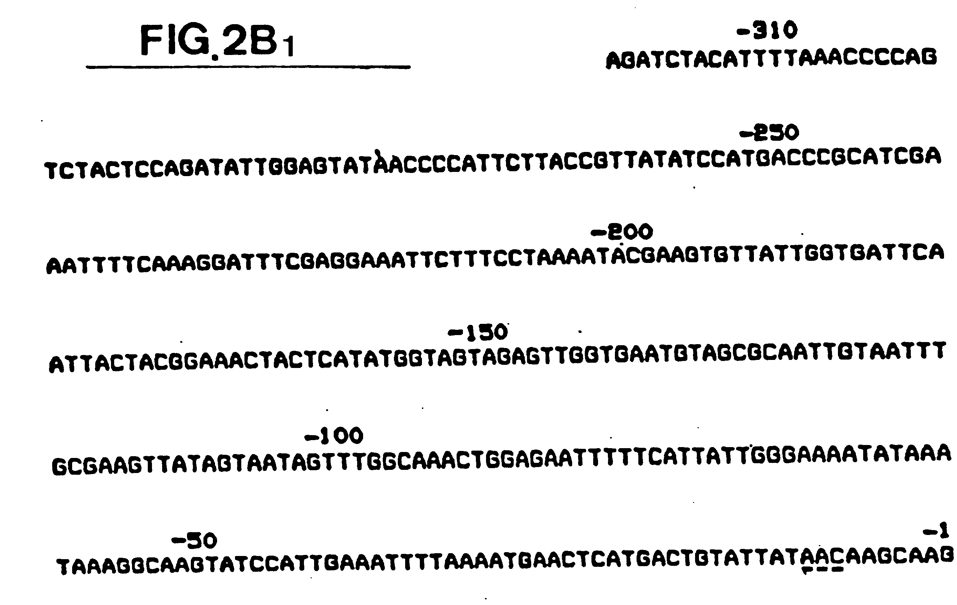

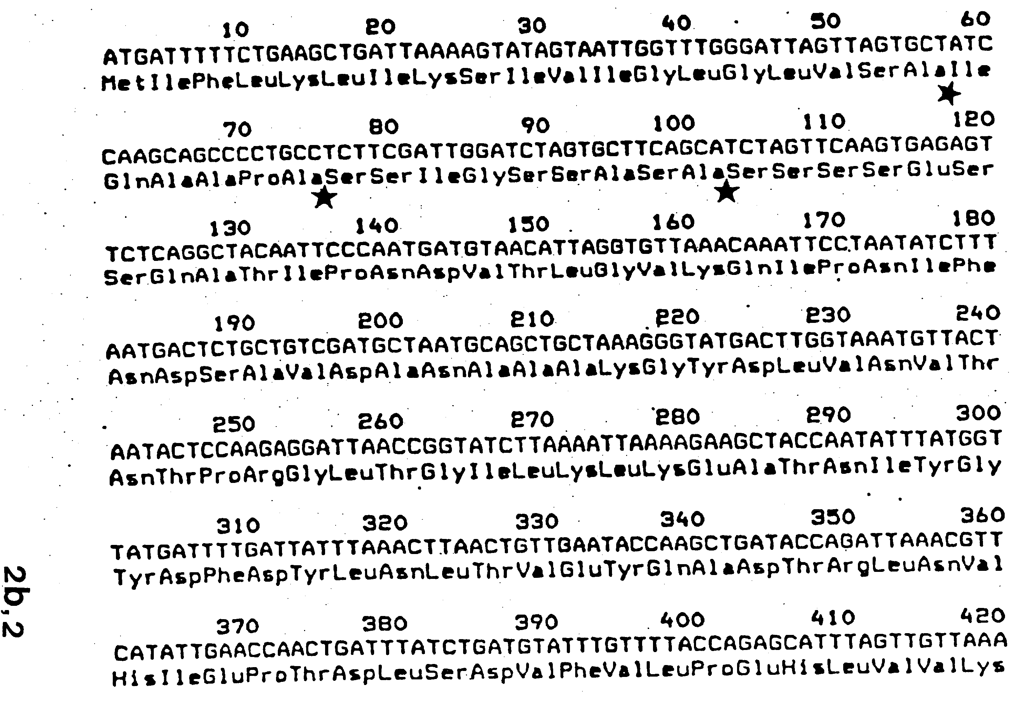

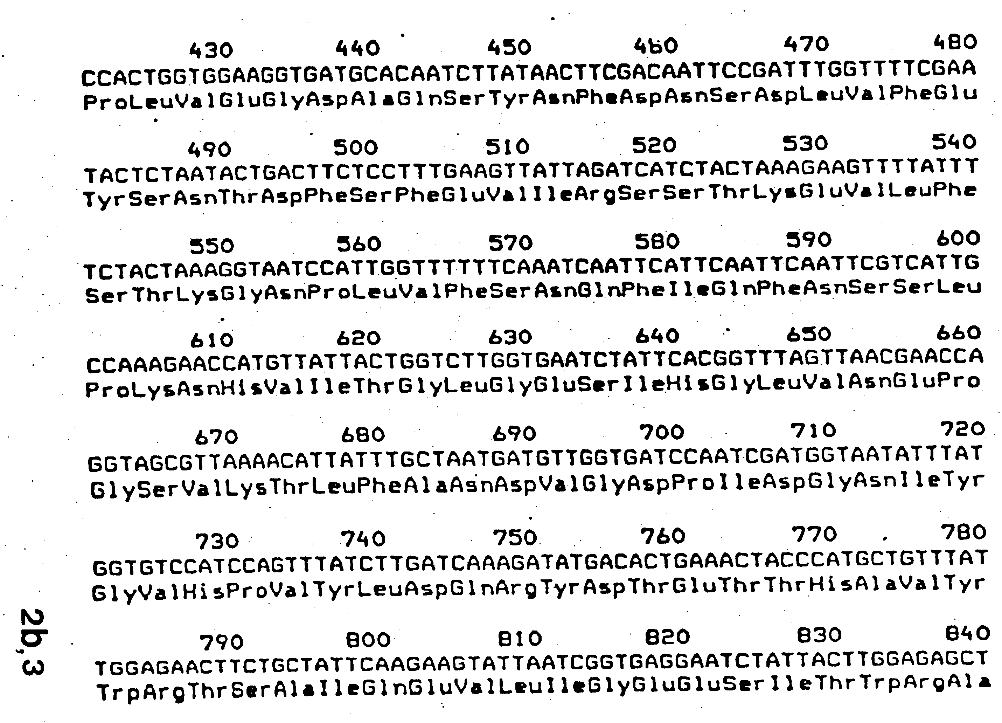

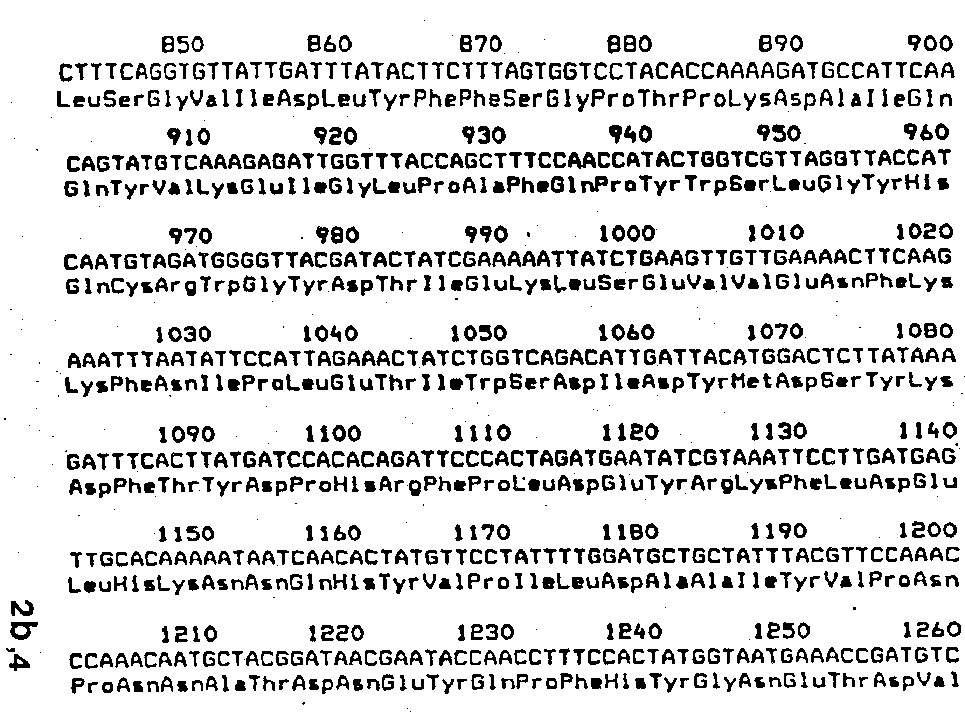

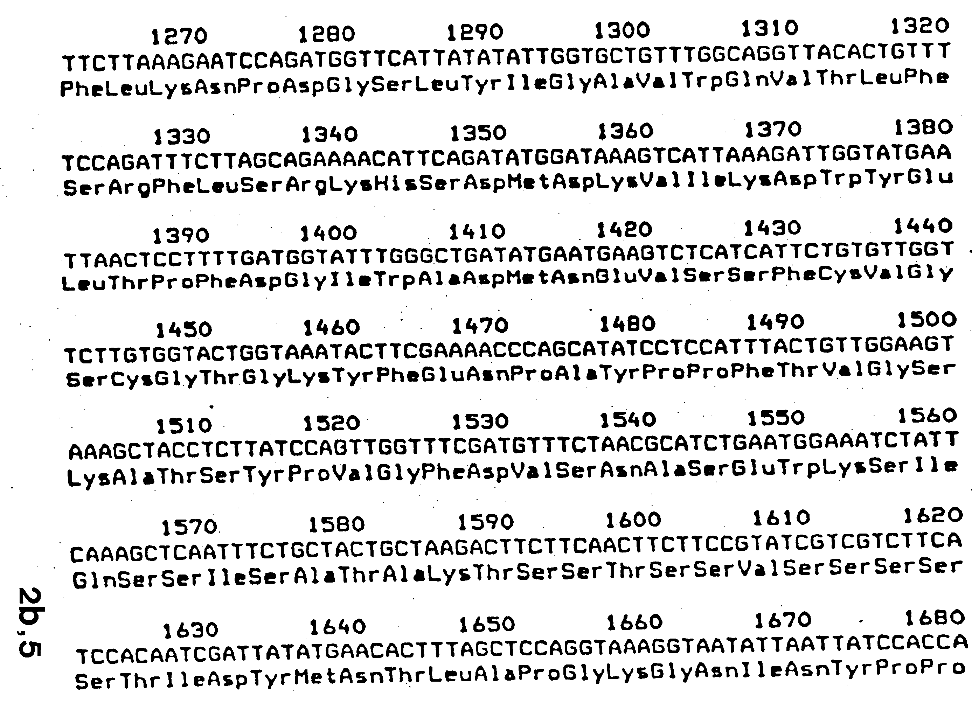

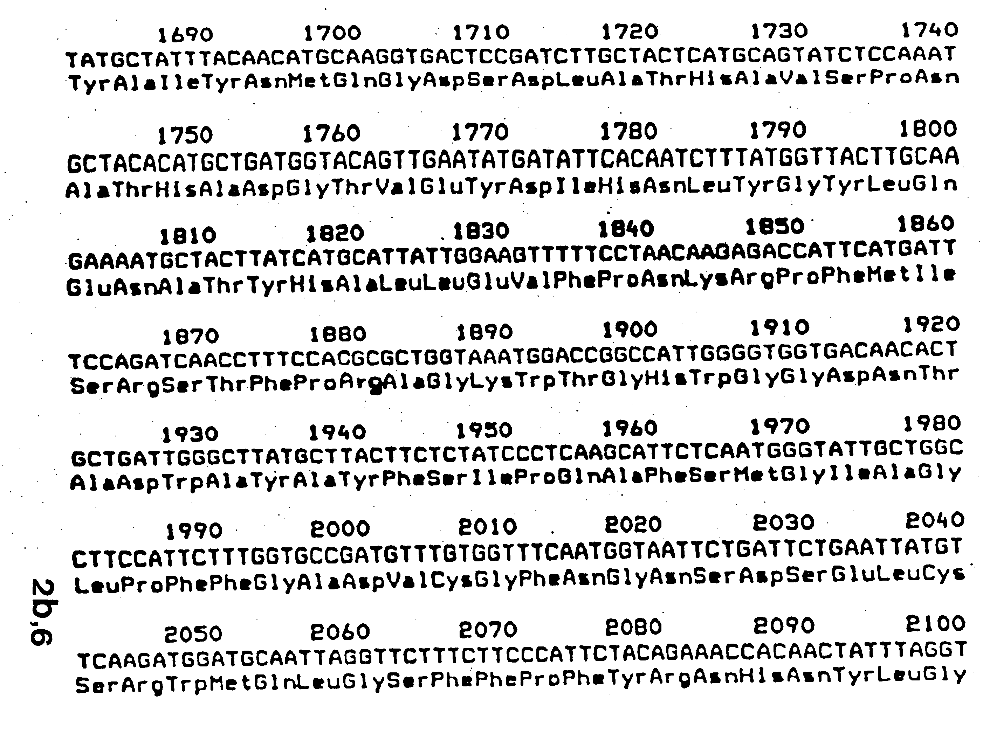

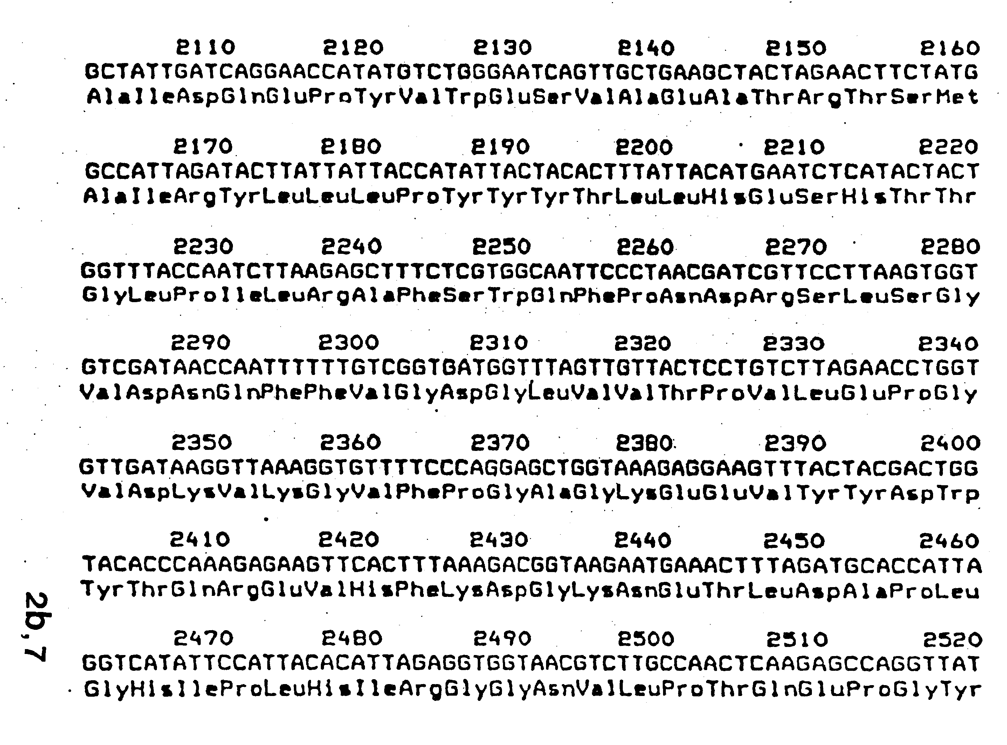

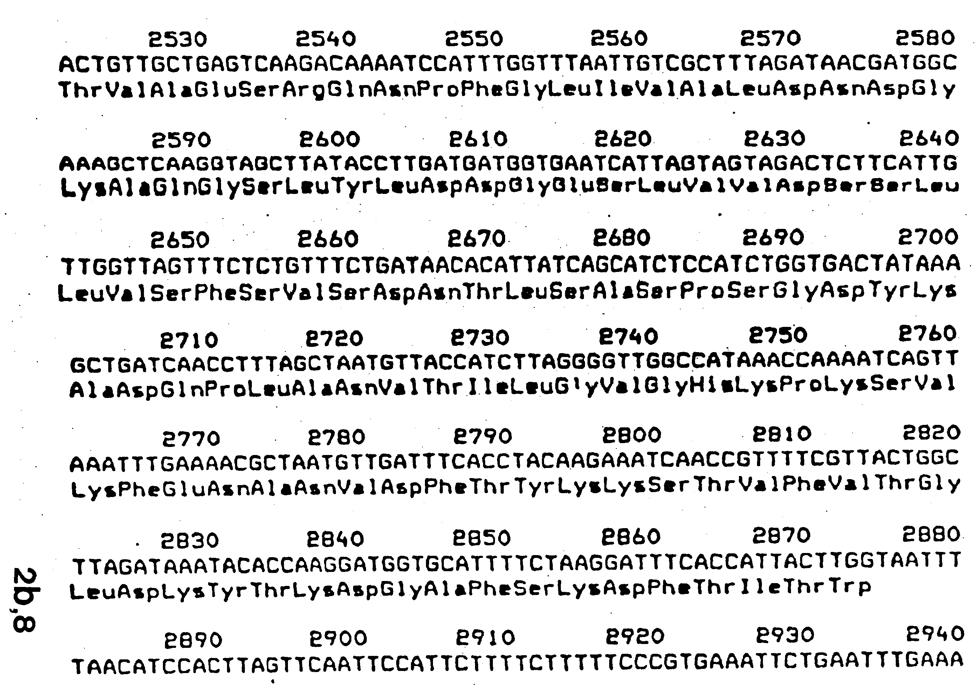

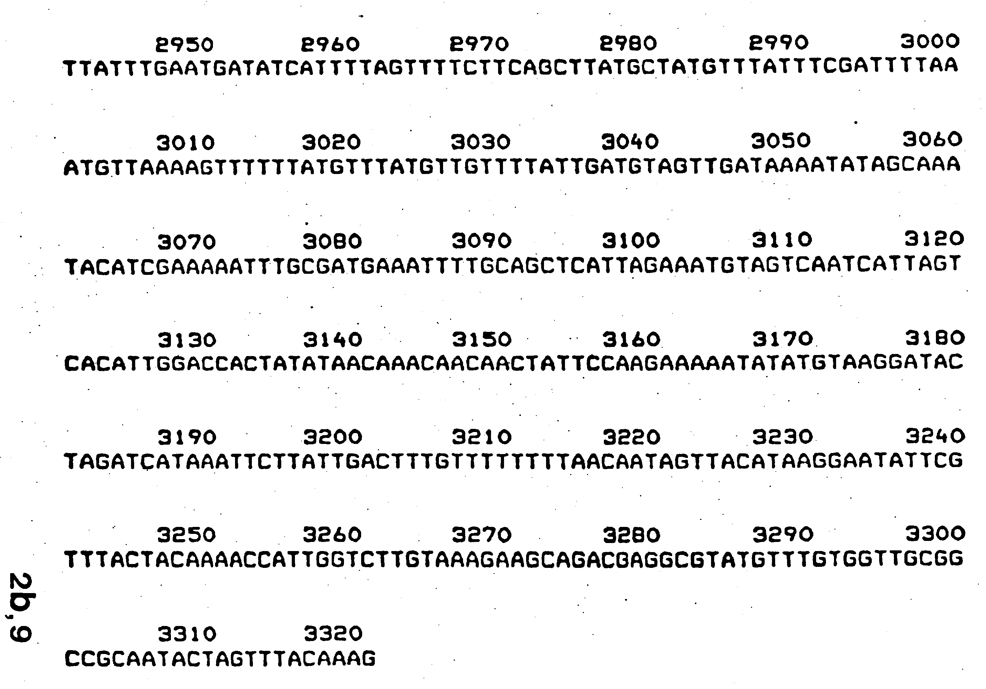

The N-termini of the deduced amino acid sequences of the α-amylase gene (Fig.1B) and glucoamylase gene (Fig.2B) exhibit the features of typical leader sequences of secretory proteins (Von Heijne 1983, Perlman and Halvorsson 1983). The N-terminal amino acids of mature α-amylase secreted by S. occidentalis are determined to be AspValser. This indicates processing between Arg33 and Asp31 which is unusual and not according to the Von Heijnes model of site specificity for signal peptidase cleavage. This putative cleavage site was confirmed also for α-amylase expressed in S. cerevisiae, Pichia stipidis, K. lactis, S. pombe and H. polymorpha. However, in these yeast species processing is also found to occur between Ala 25 and Gln 26, resulting in the N-terminal sequence GlnProIle.

α-amylase isolated from a culture of S. occidentalis migrates as a single band of 55 kDa on SDS polyacrylamide gels: following Endoglucosidase H treatment and subsequent gel electrophoreses a single band of 54 kDa is observed, indicating that only one of the two potential N-glycosylation sites of the protein is used for glycosylation. It is noteworthy that high mannose glycosylation, as very often found by proteins secreted in S. cerevisiae is not accompanying secretion in S. occidentalis.

### IDENTIFICATION OF THE TRANSCRIPTION INITATION SITES AND PROMOTER FUNCTION OF THE α-AMYLASE AND GLUCOAMYLASE GENE.

By S1 mapping of polyA-mRNA (Sharp et al., 1980) the transcription initiation site of the α-amylase gene could be localised to be in the region of -42 to -30 (indicated by an interrupted line above the relevant sequence in Fig.1B) and the transcription initiation site of the glucoamylase gene was determined to be in the region -7 to -10 (underlined in Fig.28).

### CONSTRUCTION OF AN α-AMYLASE - GLUCOAMYLASE HYBRID GENE

In order to facilitate use of the α-amylase promoter and glucoamylase structural gene in further constructions additional restriction sites were inserted in front of the translation initiation codon of both genes using oligonucleotide directed mutagenesis as described in the AMERSHAM protocol. Correct mutagenesis was verified by sequencing according to Sanger (1977). The boundaries between the original Schwanniomyces genes and the inserted polylinker are shown below :

α-amylase :

** *

5' ..TAAAATAAAAGCTCGAGATGAGATTT...3'.

XhoI

Glucoamylase :

* ****** **

5' ..CTCATGACTGTGTCGACGGATCCAAGATGATTTTT....3'.

SalI BamHI

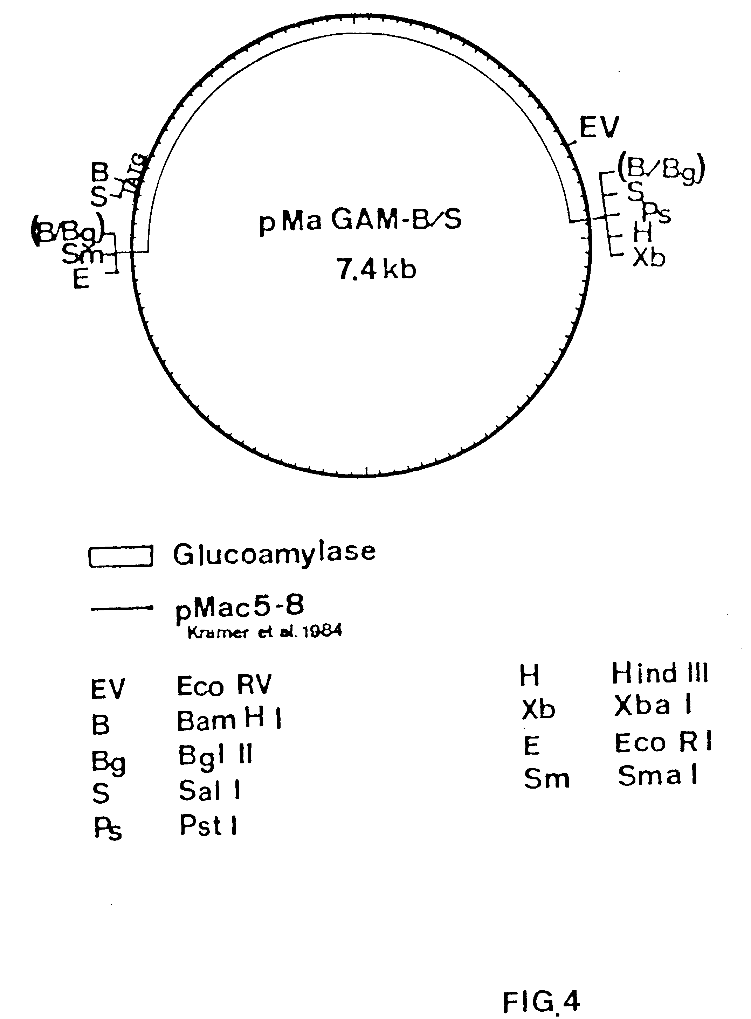

The manipulations resulted in plasmids M13αXho (Fig.3) and pMaGAM-B/S (Fig.4).

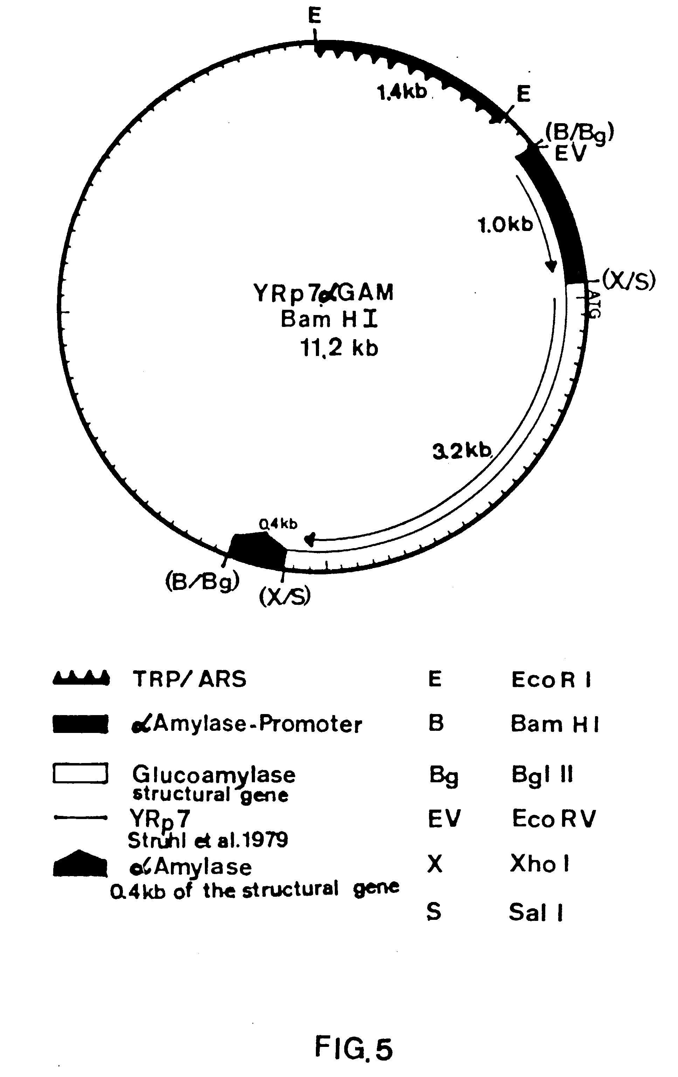

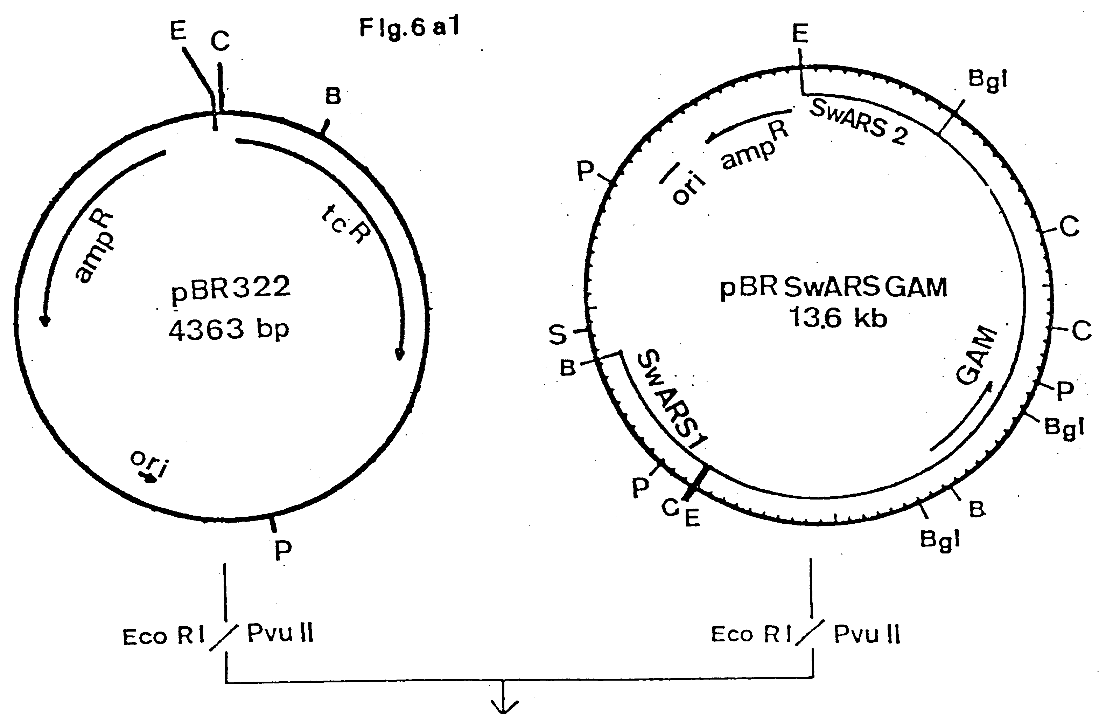

Thus, both the α-amylase promoter and the structural glucoamylase gene can be isolated from plasmid M13α Xho and pMaGAM-B/S as suitable DNA fragments. The GAM gene was isolated from pMaGam B/S as a 3.2 kb SalI fragment and ligated into the Xhoi site of M13αho. The BglII fragment harbouring the α-amylase promoter/GAM gene fusion was ligated into the single BamHI site of the S. cerevisiae vector YRp7 (Struhl et al., 1979) resulting in plasmid YRp7αGAM (Fig 5). S. cerevisiae strain YNN27 was transformed with this plasmid selecting for TRP prototrophy. Transformants secreted active glucoamylase into the culture supernatant (50 mU/ml) indicating that the α-amylase promoter fragment can direct expression and secretion of foreign proteins.

### SECRETION OF α-AMYLASE AND GLUCOAMYLASE IN DIFFERENT YEASTS USING SUITABLE PROMOTERS.

Expression of the α-amylase and glucoamylase gene and secretion of the gene products can be detected in K.lactis under control of the promoter of the α-galactosidase (LAC4) gene (Breunig et al., 1984). Expression of the α-amylase and glucoamylase gene and secretion of the both proteins can also be detected in S. pombe under control of the ADH1 promoter (Russel et al., 1983) or of the GAL1/10 promoters (Johnston and Davis 1984).
After fusion with the GAL1/10 promoter (Johnston and Davis 1984), DHAS promoter (Janowicz et al., 1985), MOX promoter (Ledeboer et al., 1985) or FMDH promoter (EP 87 110 417.0) expression of the α-amylase and glucoamylase gene and secretion of both proteins can be detected in Hansenula polymorpha.

### EXPRESSION AND SECRETION STUDIES IN S. OCCIDENTALIS USING THE CELLULASE ENDOGLUCANASE D (EGD) OF CLOSTRIDIUM THERMOCELLUM AS A MODEL FOR FOREIGN GENE EXPRESSION.

As a first example for the expression of a foreign gene in S. occidentalis, the celD gene from C. thermocellum (Millet et al., 1985, Joliff et al., 1986b), which codes for a thermostable cellulase, namely an endoglucanase (EGD) was used. Advantages in using this system are :
- specific reaction of EGD, which can easily be monitored
- a rapid qualitative assay for colonies with EGD activity (see material and methods)
- availability of antibodies against EGD for specific detection of translation products.

### CHARACTERISATION OF THE GLUCOAMYLASE PROMOTER AFTER FUSION WITH THE celD GENE

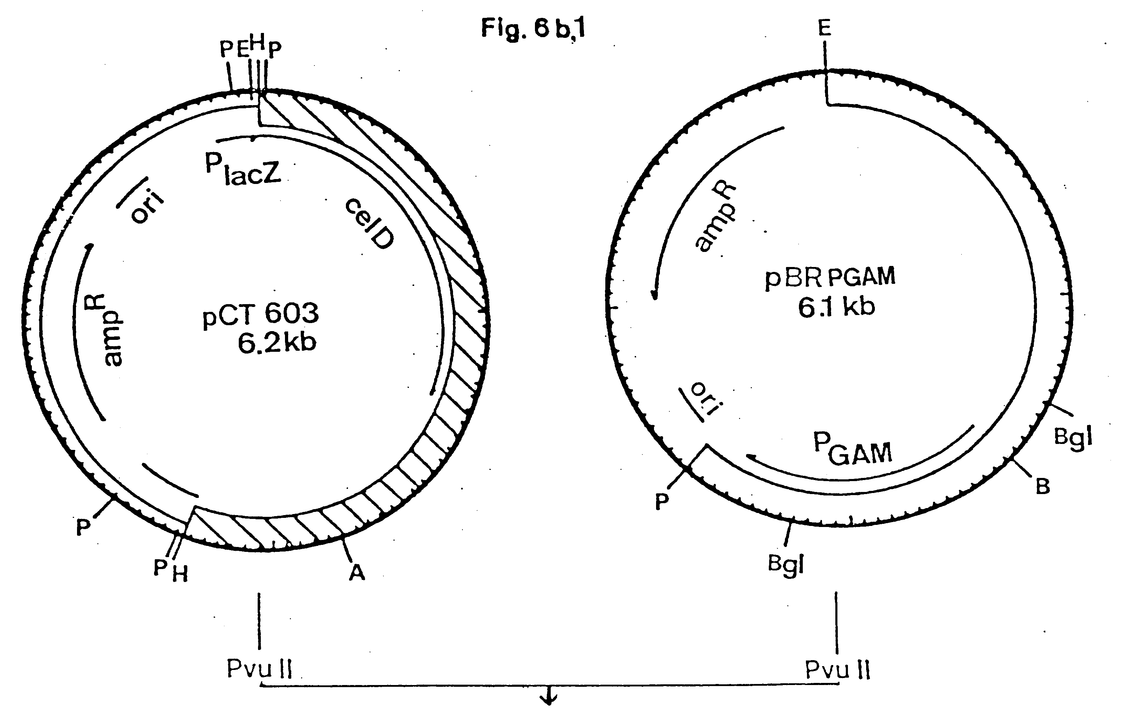

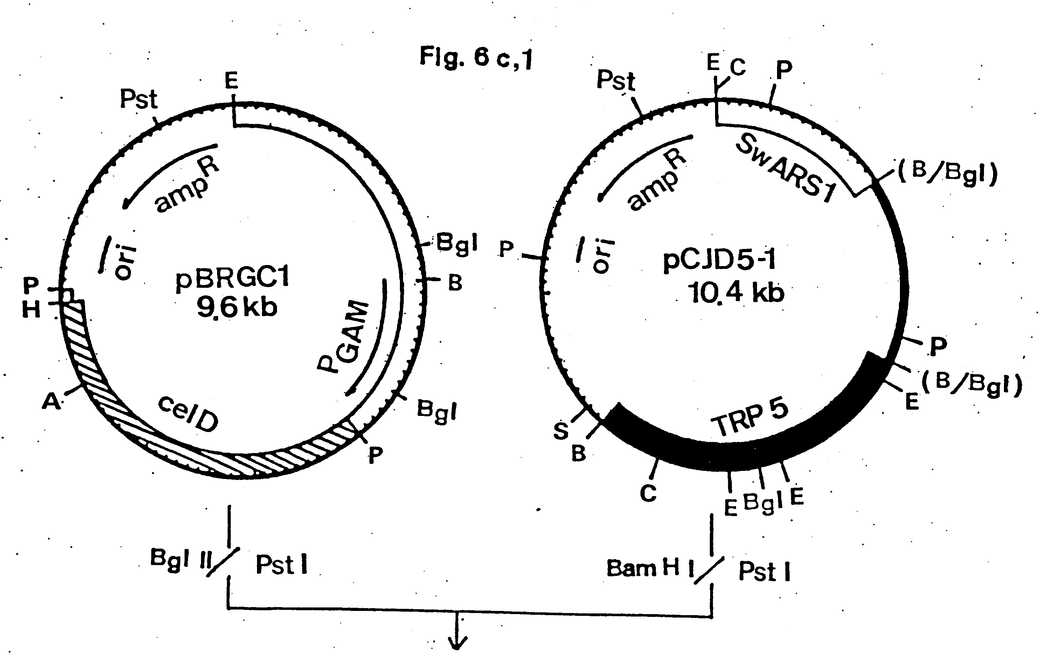

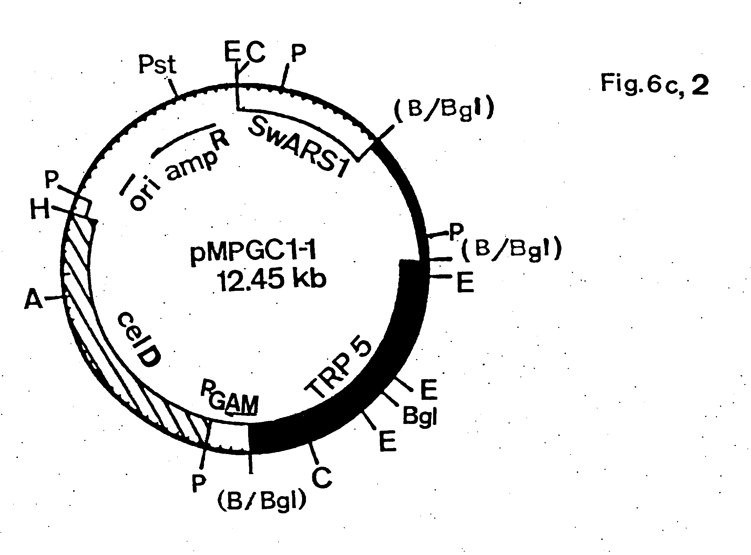

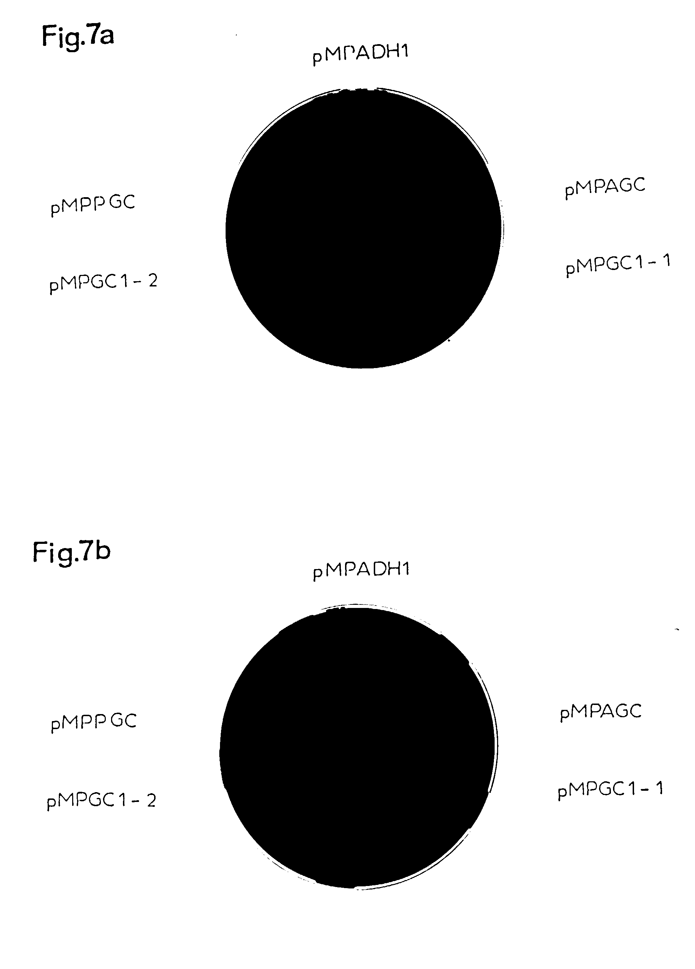

Plasmids were constructed containing a replicon from S. occidentalis (SwARS1) (EP 87110370.1) the TRP5 gene from S. cerevisiae (Zalkin and Yanowski,1982) as a selective marker and, in addition, a GAM/celD gene fusion under the control of different promoters. In the first step the 4.0 kb EcoRI-PvuII fragment from plasmid pBRSwARSGAM (Fig. 6a) was isolated and inserted into the 2296 bp pBR322 EcoRI-PvuII fragment, containing the amp gene and the bacterial origin, resulting in plasmid pBRPGAM (Fig.6a). In addition to the pBR322 sequence this plasmid carries 3.6 kb from the 5′ non coding region of the GAM gene and the first 208 bp coding for the N-terminal part (including the signal sequence) of the glucoamylase. A 3.4 kb PvuII fragment from plasmid pCT6O3 (Joliff et al., 1986) containing the coding region of the celD gene missing the 5′ 111 bp was inserted into the PvuII single site of pBRPGAM resulting in pBRGC1 (Fig.6b). E. coli transformed with this plasmid results in weak Carboxymethylcellulase (CMCase) activity, analysed by congo red staining (see material and methods) indicating that the glucoamylase promoter is slightly expressed in E. coli. For the construction of a S. occidentalis expression vector the plasmid pCJD5-1 (EP 87110370.1) was cleaved with BamHI/PstI and ligated with the 5.5 kb BglII-PstI fragment or 6.5 kb BamHI-PstI fragment from pBRGC1, resulting in pMPGC1-1 (Fig.6c) and pMPGC1-2 (Fig.6d), respectively. Both plasmids share an in frame fusion of GAM/celD but differ in the length of the 5′ upstream region of the GAM gene. S. occidentalis strain NGA58 (trp5 ade) isolated after UV mutagenesis of strain NGA23 was transformed with these plasmids and transformants selected for tryptophan prototrophy and analyzed by means of congo red assay for cellulase activity. Transformants produced active EGD when grown under inducing conditions in 2% maltose (Fig 7a). By analysis with the congo red assay no activity could be detected under repressed conditions in 4% glucose (Fig. 7b). NGA58:pMPGC1-1 and NGA58:pMPGC1-2 transformants secrete active EGD into the culture supernatant when grown in maltose under induced conditions. The intact or complete glucoamylase promoter resides on plasmid pMPGC1-2 as shown by 20 times higher activity compared to the shorter promoter fragment in gene fusion of pMPGC1-1 (table 1). However the 0.3 kb promoter fragment still harbours regulatory units which lead to catabolite repression by glucose (Fig.7b). Comparison of the N-terminal GAM sequence to the signal peptidase cleavage sites of several other secretory proteins (Von Heijne 1983) reveals three potential signal peptidase cleavage sites after the amino acid residues Ala 19, Ala 25 and Ala 34. For determination of the N-terminal amino acid of the mature glucoamylase the protein was isolated for N-terminal amino acid sequencing. However the N-terminus is blocked and therefore could not be sequenced. To confirm the correct cleavage site of the GAM signal sequence in S. occidentalis, the regions between the potential signal peptidase cleavage sites and the PvuII gene fusion site are deleted in the GAM/celD fusion by in vitro mutagenesis to study the influence on secretion of active EGD.

### STUDIES OF THE USE OF SUITABLE S. CEREVISIAE PROMOTERS IN THE EXPRESSION OF FOREIGN GENES IN S. OCCIDENTALIS.

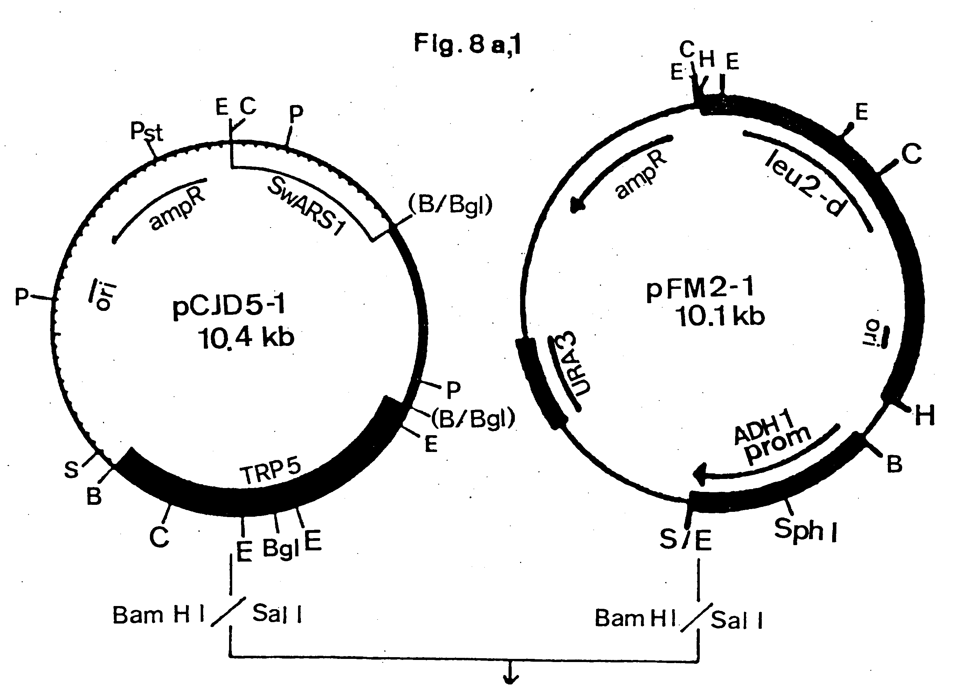

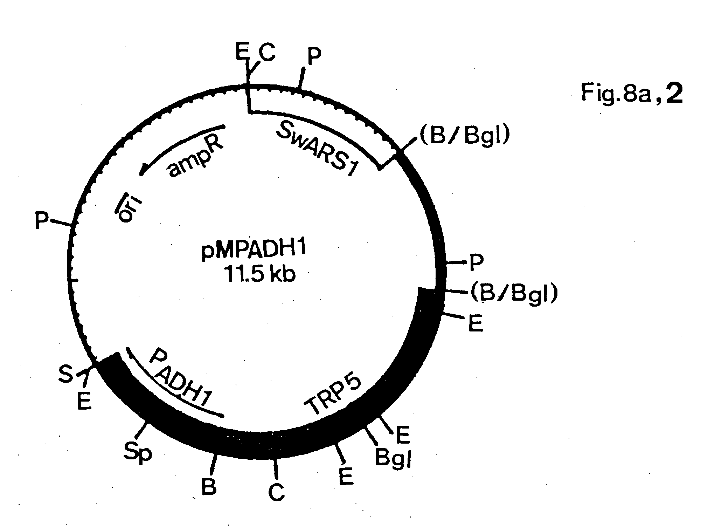

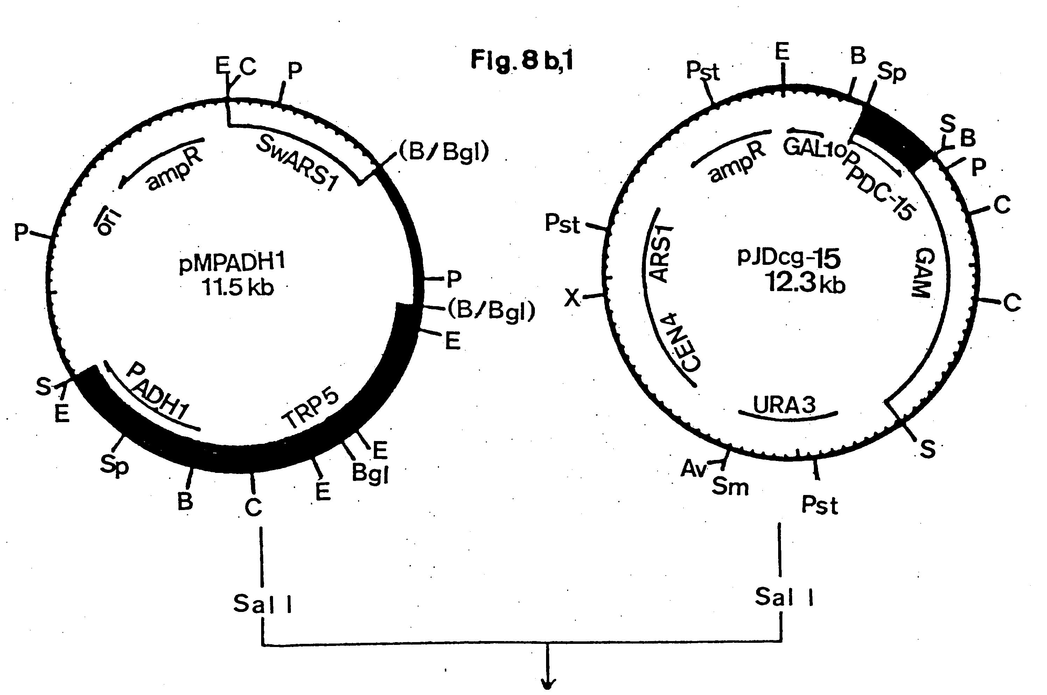

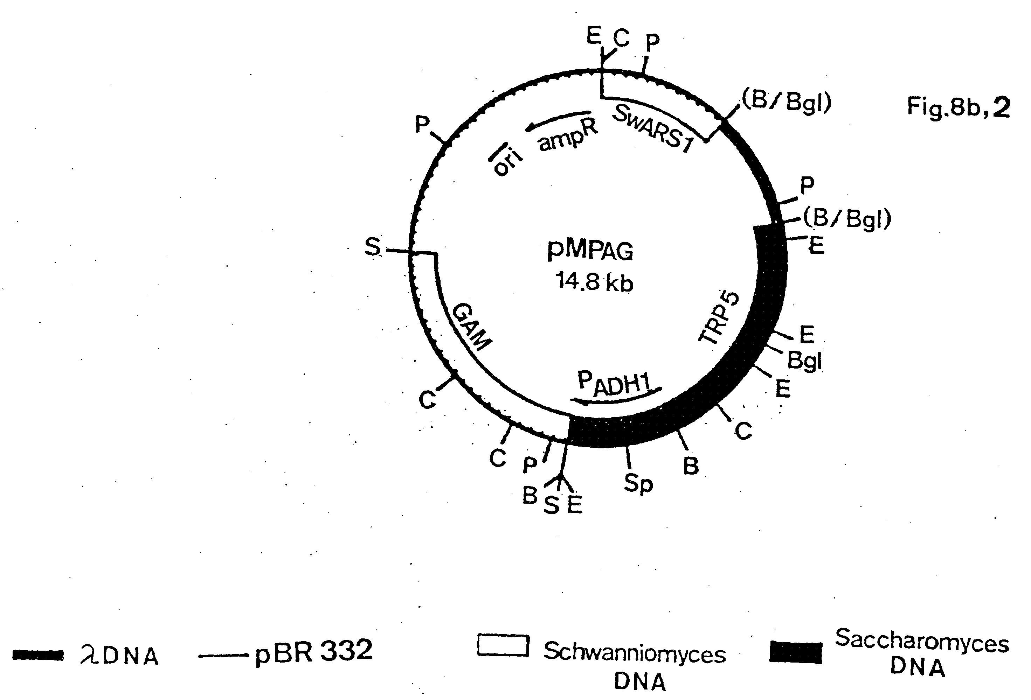

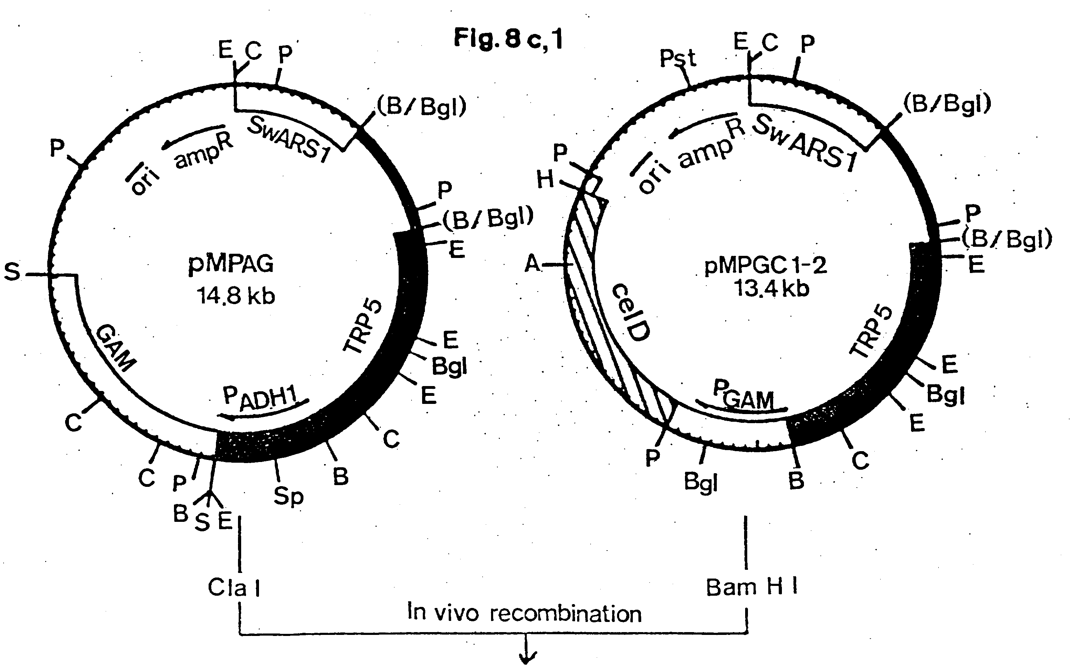

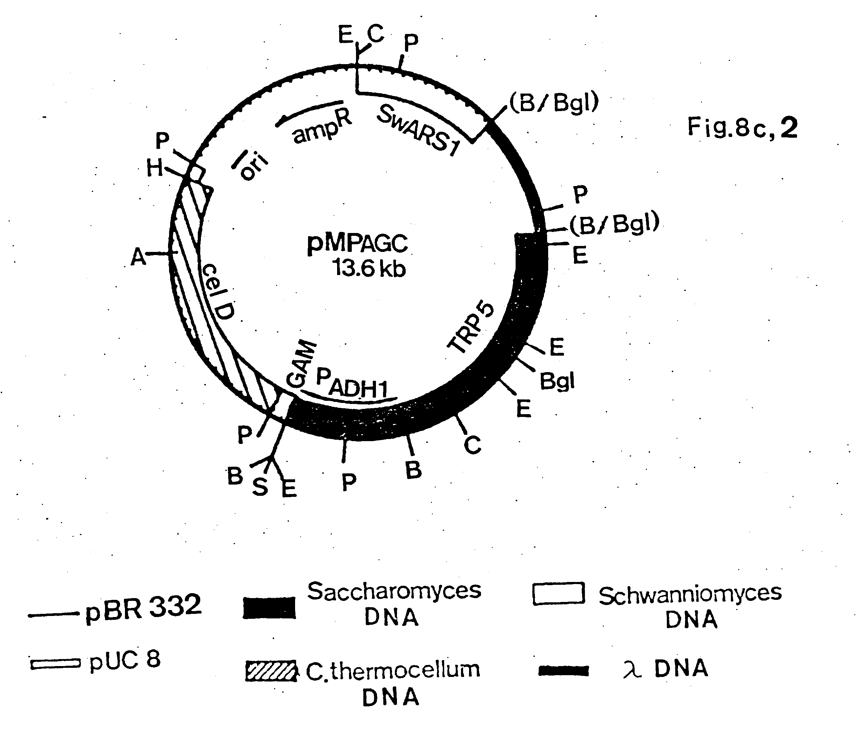

The ADH1 promoter (Hitzeman et al., 1981) was isolated as a 1.45 kb BamHI-SalI fragment from plasmid pFM2-1 (Müller et al., 1987) and ligated into plasmid pCJD5-1 (EP 87 110370.1) after cleavage with BamHI and SalI resulting in pMPADH1 (Fig.8a). The GAM gene was excised as a SalI fragment from pJDcg-15 (see below) and inserted into the single SalI site of pMPADH1 (Fig. 8b) resulting in plasmid pMPAG (Fig. 8b). By the means of in vivo recombination (Ma et al., 1987) the GAM promoter from pMPGC1-2 (Fig 6d.) was replaced by the ADH1 promoter of pMPAG leading to plasmid pMPAGC (Fig. 8c). Plasmid pMPGC1-2 was linearized with BamHI; the unique BamHI site of pMPGC1-2 is localized between the TRP5 coding sequence and the glucoamylase promoter. The 2.75 kb ClaI fragment of pMPAG containing the ADH1 promoter shares homologies to the TRP5 gene as well as to the GAM coding region up to position +208. For in vivo recombination NGA58 was transformed with BamHI linearized pMPGC1-2 together with approx. 10 fold excess of ClaI fragment using the transformation protocol of Ito et al., 1983.

Recombinants were screened for their ability to hydrolyze CMC on 4% glucose. Under these conditions celD expression is repressed in transformants harbouring recircularized pMPGC1-2. From 146 transformants 7 colonies expressing cellulase in the presence of 4% glucose were isolated. NGA58 containing the "in vivo" constructed plasmid pMPAGC expresses active EGD constitutively (see Fig. 7). This result demonstrates that the newly constructed plasmid pMPADH1 serves as a vector for foreign gene expression.

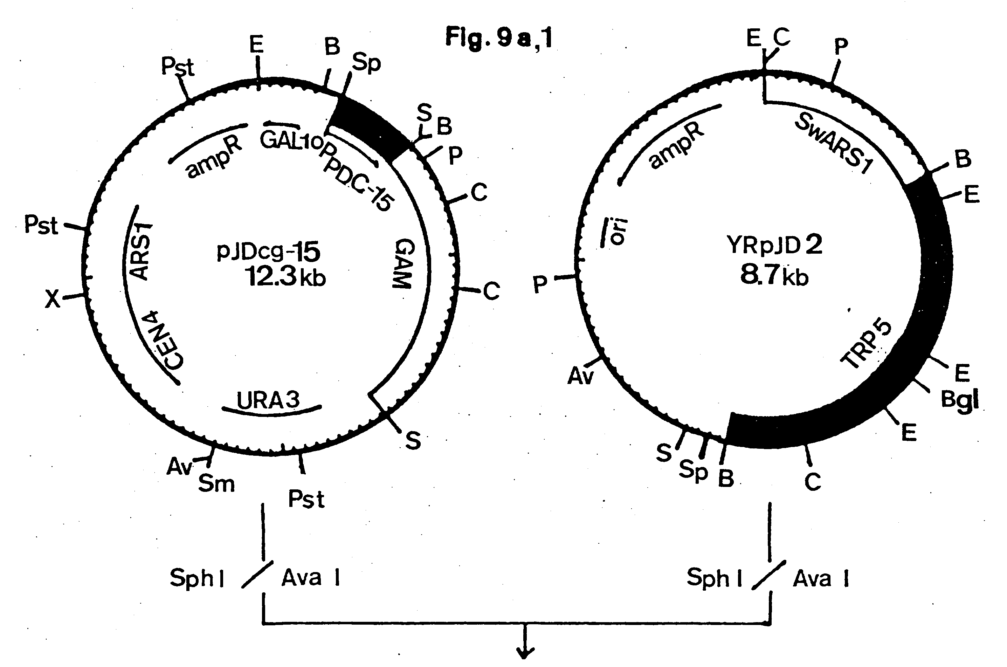

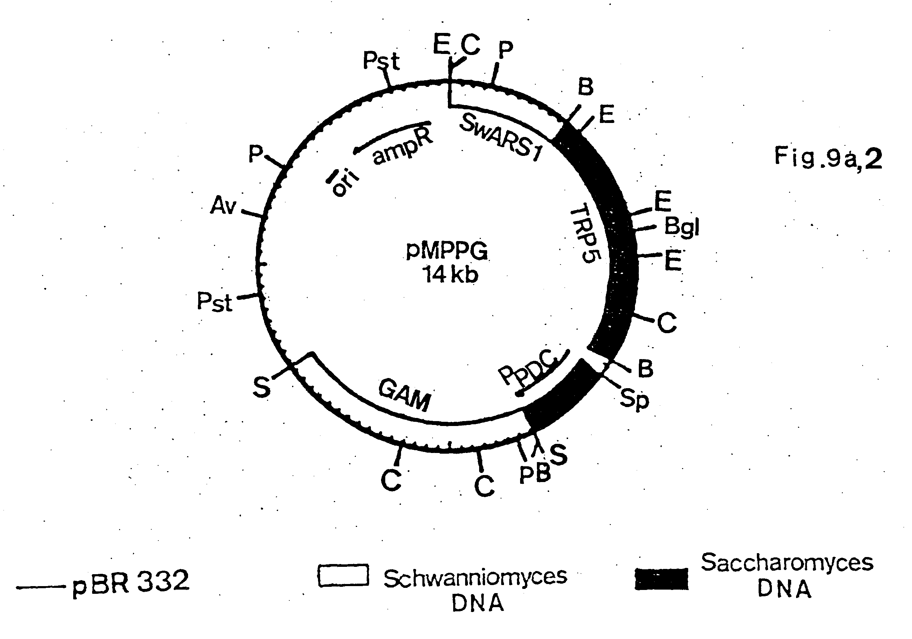

In another experiment a recombinant plasmid was constructed by in vivo recombination to study the expression of the GAM/celD gene fusion under control of the PDC promoter (Das and Hollenberg, 1982). Therefore the 6.0 kb SphI-AvaI fragment from plasmid pJDcg-15 (EP 87 110370.1) carrying the glucoamylase gene fused to the PDC promoter was subcloned into the respective restriction sites of YRpJD2 (EP 87 110370.1) resulting in plasmid pMPPG (Fig. 9a).

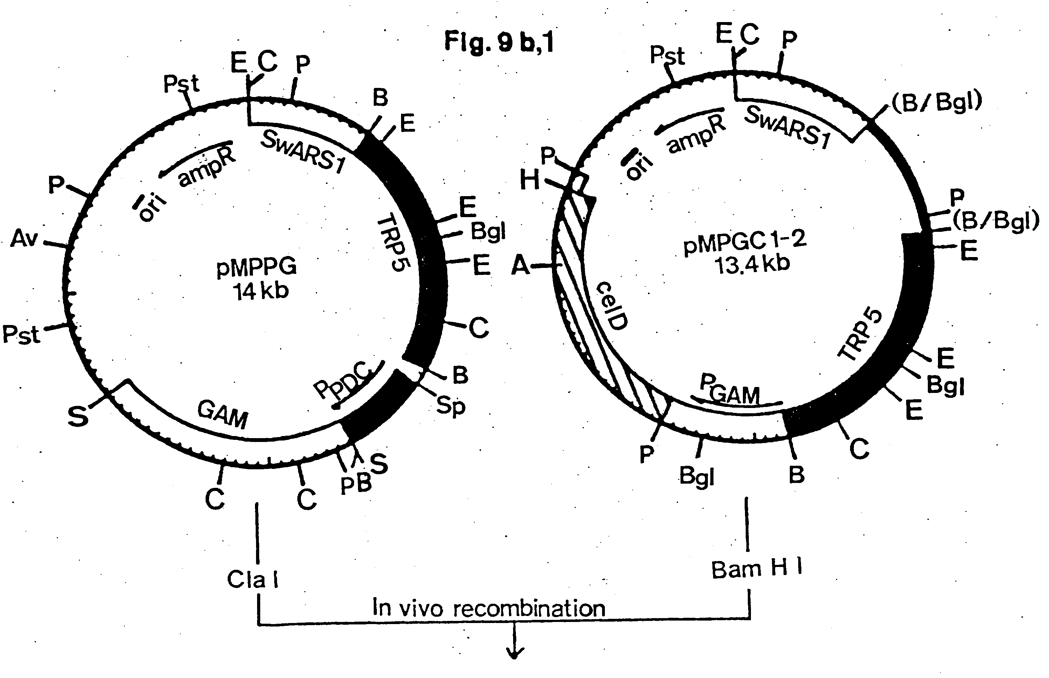

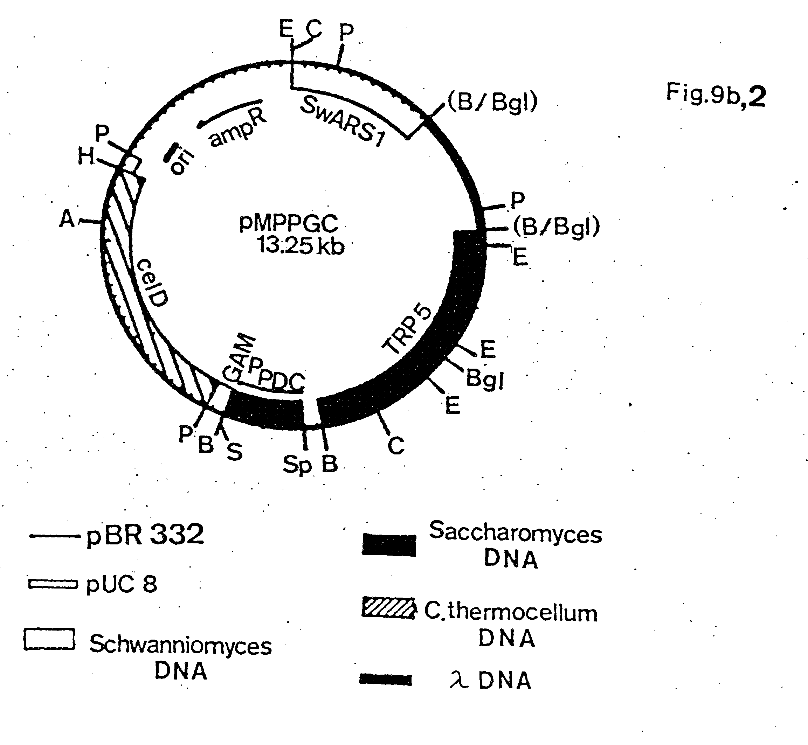

For in vivo recombination pMPPG was cleaved with ClaI and used together with BamHI linearized pMPGC1-2 for transformation of NGA58. Homologous recombination results in plasmid pMPPGC (Fig. 9b) harbouring a GAM-celD fusion under control of the PDC promoter. This construction leads to an expression in Schwanniomyces occidentalis NGA58 inducible by glucose and partly repressable by maltose (Table 1). Transformants were analysed for expression of active EGD using the qualitative congo red assay (Fig. 7). The results indicate that the 0.3 kb GAM promoter fragment leads to regulated expression, however less efficient that the 1.3 kb fragment (for comparison see table 1 and Fig. 2A). Expression under the ADH1 promoter leads to efficient constitutive expression. The expression level is comparable with that of induced expression directed by the 1.3 kb GAM promoter.

The fact that plasmids can be constructed by in vivo recombination is a proof for homologous recombination and hence offers the possibility for stable site directed integration into the genome of S. occidentalis, a prerequisite for the industrial production of foreign proteins.

The expression of the α-amylase gene and glucoamylase gene and secretion of the both gene products in S. occidentalis could also be achieved under control of the phosphoglycerate kinase (PGK) promoter (Dobson et al., 1982), glyceraldehyde-3-phosphate dehydrogenase (GAPDH) promoter (Holland and Holland 1979), copperchelatin (CUP1) promoter (Karin et al., 1984, Butt et al., 1984) and alcoholdehydrogenase 2 (ADR2 or ADH2) promoter (Russell et al., 1983, Beier et al., 1985) or galactokinase (GAL1/10) promoter (Johnston and Davis, 1984).

### Construction of pJDcg-15 :

The GAM gene was isolated as a 3.2 kb BamHI-SalI DNA fragment from plasmid pMaGAM B/S (Fig.4) and inserted into the respective restriction sites of plasmid pBM272 Johnston & Davis, 1984). The PDC promoter was isolated from plasmid pCP202 (Kellermann & Hollenberg, 1988) as a SalI-SphI fragment and ligated into the respective single sites of vector YRp7 (Struhl et al., 1979). To be able to isolate the PDC1 promoter as a BamHI fragment the 3.2 kb SalI fragment of PMaGAM B/S containing the GAM gene was fused to the PDC promoter via the single SalI site. The resulting BamHI fragment containing the PDC-promoter was ligated into the single BamHI site of pBM272 containing the GAM gene. The final plasmid is called pJDcg-15 (Fig. 8b).

### EXPRESSION OF HUMAN GRANULOCYTE/MACROPHAGE COLONY STIMULATING FACTOR (hGM-CSF)

The gene coding for hGM-CSF (Cantrell et al., 1985) was obtained from British Biotechnology Ltd. as a HindIII-EcoRI fragment. Position +52 of hGM-CSF gene was fused to position + 102 of the GAM signal sequence under control of the ADH1 or GAM promoter. This fusion results in a hybrid protein in which the endopeptidase cleavage site of the GAM signal sequence is linked with a Met included in the synthetic gene offered by British Biotechnology Ltd., followed by the first amino acid of mature hGM-CSF (Ala 18). This fusion was inserted into a S. occidentalis expression vector and transformed into NGA58, Western blot analysis revealed secretion of hGM-CSF protein.

### EXPRESSION OF INTERLEUKINE-2

The gene coding for IL-2 (Taniguchi et al., 1983) was obtained from British Biotechnology Ltd., as a EcoRI-HindIII fragment. Position +61 of IL-2 gene was fused to position + 102 of the GAM signal sequence under control of the ADH1 or GAM promoter. This fusion results in a hybrid protein in which the endopeptidase cleavage site of the GAM signal sequence is linked with a Met included in the synthetic gene offered by British Biotechnology Ltd., followed by the first amino acid of mature IL-2 (Ala 21). This fusion was inserted into a S. occidentalis expression vector and transformed into NGA58. Western blot analysis revealed secretion of IL-2 protein.

### ISOLATION OF AN ADDITIONAL SwARS ELEMENT AND A NEW SELECTABLE MARKER

A new SwARS sequence (SwARS2), localized downstream of the coding region of the GAM gene, could be identified. To analyze the function of SwARS2 a vector was constructed containing the Trps gene and SwARS2 in pBR322, resulting in pMPTS2-A and pMPTS2-B, respectively (Fig. 10). For the construction of pMPTS2-A and pMPTS2-B the 5.5 kb BamHI - BgII fragment of pBRSwARSGAM, constructed by insertion of the EcoRI-fragment shown in Fig.2a into the single EcoRI site of plasmid pBRSwARS1, (disclosed in EP 87 110370.1), was isolated and ligated with 3.2 kb BamHI TRP5 fragment obtained from YRpJD2. High frequency transformation of S. occidentalis is obtained using this plasmid.

As an additional selectable marker the LEU2 gene of S. occidentalis was isolated. The LEU2 gene, coding for isopropyl malate dehydrogenase, was cloned by functional complementation of the mutant AH22 (his4leu2) (Hinnen et al., 1978) of S. cerevisiae by transformation with a cosmid genomic library from S. occidentalis.

The LEU2 gene was found to reside on a 9 kb EcoRI fragment of Schwanniomyces genomic DNA; it's function could be abolished by insertion of the TRP5 gene of S. cerevisiae. The disrupted LEU2 gene was integrated into the genome of NGA58 by homologous recombination resulting in a new strain NGA581 (TRP5, LEU2, and ADE). An analogous experiment with the cloned HIS4 gene of S. occidentalis (EP 87 110 370.1) led to a strain NGA58h (TRP5, HIS4, ADE).

### IMPROVED EXPRESSION OF HETEROLOGOUS GENES USING THE T7 RNA POLYMERASE AND T7 PROMOTER AND TERMINATER FOR THE EXPRESSION OF FOREIGN GENES IN S. OCCIDENTALIS

T7 RNA polymerase (Fuerst et al., 1986) can be expressed in S. occidentalis after fusion of the structural gene (Dunn and Studier, 1984) using the BamHI restriction site near the translation initation codon with suitable promoters asα-amylase, GAM (0.3 kb fragment) or PDC and homologous integration into the genome via any homologous cloned gene, e.g. HIS4, LEU2, AMY1 and GAM. A suitable fragment, containing the fusion flanked by homologous cloned sequences can be isolated and used together with pCJD5-1 (Fig. 6c) for co-transformation of NGA58, selecting on YNB containing 0.5% casaminoacids. Colonies containing the integrated T7 RNA polymerase gene can be identified by Southern analysis after isolation of transformants mutated in the target gene.

For the expression of foreign genes a S.occidentalis expression vector was constructed containing SwARS2, the ColE replication origin of E.coli, a suitable selective marker for S. occidentalis (e.g. TRPS, HIS4, LEU2) and a cassette containing the T7 polymerase promoter and terminator sequences isolated from plasmid pAR2529 (Fuerst et al., 1986), separated by the polylinker sequence from M13mp19. To study the efficiency of the T7 system we expressed a GAM/celD fusion (GAM signal sequence and structural celD gene obtained as a BamHI-Asp 718 fragment from pMPGC1-2) under control of the T7 promoter by insertion into the BamHI-Asp718 site of the polylinker sequence. Using this system active cellulase is secreted into the culture medium.

### MATERIALS AND METHODS

### 1. The microorganisms used are as follows :

- Schwanniomyces occidentalis: NGA23 : (DSM 3792)
- Schwanniomyces occidentalis: NGA58
- Kluyveromyces lactis: SD11 : (DSM 3795)
- Schizosaccharomyces pombe: LEU1-32, HIS5-303 (DSM 3796)
- Saccharomyces cerevisiae: YNN27 (URA3-52, TRP1-298, GAL2)

The Escherichia coli strain used is as follows E. coli HB101 F-. hsd S20 (r_{B}- m_{B} -), recA13, ara-14, proA2, lacY1, galK2, rpsL20 (Sm^{r}), xyl-5, mtl-1, supE44)

### 2. Culture media :

Schwanniomyces occidentalis strain NGA58 (isolated by UV mutagenesis of strain NGA23) was grown at 30°C in YNB (0.67% yeast nitrogen base without amino acids) supplemented with appropriate amino acids and with 2% of soluble starch when indicated.

S. cerevisiae strain YNN27 (URA3-52, TRP1-289, GAL2) (Stinchomb et al 1980); K. lactis strain SD11 TRP1 (Das and Hollenberg, 1982); S. pombe strains LEU1-32, HIS5-303 (obtained from W.D. Heyer): These strains were grown under conditions as described above for Schwanniomyces occidentalis with the replacement of 2% soluble starch by 2% glucose, where indicated.

The culture media for S. occidentalis and Hansenula polymorpha were buffered with 0.2 M NaP0₄ buffer pH 6.2; those for S. cerevisiae and K. lactis were buffered with 0.1 M citrate buffer pH 6 and those for S. pombe with 0.05 M acetate buffer pH 6.

The pH optimum for the cellulase (Joliff et al., 1986a), α-amylase and glucoamylase (Wilson and Ingledew, 1982) is approx. pH 6.

E. coli HB101 (Bolivar et al., 1977) was grown in LB medium (Maniatis et al., 1982) with penicillin G (150 ug/ml).

### 3. Miscellaneous methods

Plasmid DNA was purified either by CsCl gradient centrifugation (Maniatis et al., 1982) or by rapid alkaline extraction of plasmid DNA described by Birnboim and Doly (1979).

Yeast crude extracts were prepared by the method of Schatz (1979). Yeast minilysates were prepared by the method of Sherman et al., (1983).

DNA fragments were isolated by the "low melting agarose" procedure as described by Gafner et al., (1983). Southern hybridization was carried out as described by Southern (1975).

Yeast transformation was performed according to Klebe et al. (1983) or Ito et al. (1983). The transformants or integrants were tested for expression and secretion of cellulase using either the qualitative congo red assay (Teather and Wood, 1982: halo formation on carboxymethylcellulose agar plates) or a quantitative cellulase assay according to Joliff et al. (1986a). One unit (U) is defined as the amount of enzyme that released 1 umol para-nitrophenol per min at 60°C. Western analysis was performed as described by Towbin et al. (1979).

The transformants or integrants were tested for expression and secretion of α-amylase using either a standardized α-amylase enzyme test from Merck, W. Germany or a qualitative starch degradation test (halo formation on starch agar plates after staining with iodine), as follows : in this test the rate of formation of 2-chloro-4-nitrophenol is determined by photometry at 405 nm in 0.1 M potassium phosphate at 37°C. The enzyme unit (U) is defined as the amount of enzyme catalysing the formation of 1 umol 2-chloro-4-nitrophenol per min at 37°C.

Glucoamylase activity was measured by a stop assay method:
After incubation of the sample in 10% soluble starch in 0.05M KH₂PO₄-NaOH (pH 5.0) at 50°C the amount of glucose produced is determined by the glucose dehydrogenase method (system glucose Merck). The quantity of NADH formed is proportional to the glucose concentration. The enzyme unit (U) is defined as the amount of enzyme catalysing the formation of 1 umol glucose/min at 50°C.

**TABLE 1**

| Transformant | OD_{600 nm} | EDG-activity mU/ml |
|---|---|---|
| NGA58: | 2.3 | 0.12 |
| pMPGC1-1 (0.3 kb promoter) | 5.3 | 0.73 |
| NGA58: | 2.0 | 0.53 |
| pMPGC1-2 (1.3 kb GAM promoter) | 7.9 | 1.83 |
| NGA58: | 3.0 | 0.52 |
| pMPAGC ADH1 promoter | 5.9 | 1.07 |
| NGA58: | 2.2* | 0.40* |
| pMPPGC | 4.5* | 0.64* |
| PDC1-promoter | 2.9 | 0.19 |
| | 7.2 | 0.31 |
| Transformants were grown in YNB, 0.5% Casaminoacids, 2% maltose, 20 mg/l adenine and 0.2 M NaP buffer pH 6.2 | | |

| | | |
|---|---|---|
| * : values for transformants grown in 4% glucose | | |

### LIST OF REFERENCES

Beier et al. (1985), Mol. Cell. Biol. 5, 1743-1749.
Birnboim & DolY (1979), Nuc. Acids Res. 7, 1513-1523.
Bitter et al. (1984), Proc. Natl. Acad. Sci. USA 81, 5330-5334.
Bolivar et al. (1977), Gene 2, 75-93.
Breunig (1984), NUCl. Acids Res. 12, 2327-2341.
Butt et al. (1984), Proc. Natl. Acad. Sci. USA 81, 3332-3336.
Cantrell et al. (1985), Proc. Natl. Acad. Sci USA 82, 6250-6254.
Das & Hollenberg (1982), Curr. Genet. 6, 123-128.
Dobson et al. (1982), Nucleic Acids Research 10, 2625-2637.
Dunn & Studier (1984), Proc. Natl. Acad. Sci. USA 81, 2035-2039.
Emr et al. (1983), Proc. Natl. Acad. Sci. USA 80, 7080-7084.
Fuerst et al. (1986), Proc. Natl. Acad. Sci. USA 83, 8122-8126.
Gafner et al. (1983), Embo J. 2, 583-591.
Hinnen et al. (1978), Proc. Natl. Acad. Sci. USA 75, 1929-1933.
Hitzeman et al. (1981), Nature 294, 717-722.
Holland & Holland (1979), J. Biol. Chem. 254, 5466-5474.
Ito et al. (1983) J. Bacteriol. 153, 163-168.
Janowicz et al. (1985), Nucleic Acids Res. 9, 3043-3062.
Johnston & Davis (1984), Mol. Cell. Biol. 4, 1440-1448.
Joliff et al. (1986a), Bio/Technology 4, 896-900.
Joliff et al. (1986b), NUCl. Acids Res. 14, 8605-8613.
Karin et al. (1984), Proc. Natl. Acad. Sci. USA 81, 337-341.
Kellermann & Hollenberg (1988), Curr. Genetics 14, 337-344.
Klebe et al. (1983), Gene 25, 333-341.
Kramer et al. (1984), Nucl. Acids. Res. 12, 9441-9456.
Kurjan & Herskowitz (1982), Cell 30, 933-943.
Ledeboer et al. (1985), Nucl. Acids. Res. 9, 3063-3082.
Ma et al. (1987), Gene 8, 201-216.
Maniatis, Fritsch & Sambrock (1982), Molecular cloning: A laboratory manual, Cold Spring Harbour Laboratory press, New York.
Millet et al. (1985), FEMS Microbiol. Lett. 29, 145-149.
Müller et al. (1987), Mol. Gen. Genetics 207, 421-429.
Oteng-Gyang et al. (1981), Zeitschrift allgemeine Mikrobiologie 21, 537-544.
Perlman et al. (1983), J. Mol. 167, 391-409.
Price et al. (1978), Microbiol. Rev. 42, 161-193.
Russell et al. (1983), J. Biol. Chem. 258, 2674-2682.
Sanger et al. (1977), Proc. Natl. Acad. Sci. USA 74, 5 463-5 467.
Schatz (1979), Meth. Enzymol. LVI, 40-50.
Sharp et al. (1980), in: Methods Enzymol. 65,
Sherman et al. (1983), Methods in yeast genetics in C.S.H. Laboratory, C.S.H. New York, 119.
Sills & Stewart (1982), J. Inst. Brew. 88, 313-316.
Sills et al. (1984 a), J. Inst. Brew. 90, 311-314.
Sills et al. (1984 b), APPl. Microb. Biotechnol. 20, 124-128.
Southern (1975), J. Mol. Biol. 98, 503-517.
Stinchomb et al. (1980), Proc. Natl. Acad. Sci. USA 77, 4 559-4 563.
Struhl et al. (1979), Proc. Natl. Acad. Sci. USA 76, 1035-1039.
Taniguchi et al. (1983), Nature 302, 305-309.
Teather & Wood (1982), Appl. Env. Microbiol. 43, 777-780.
Towbin et al. (1979), Proc. Natl. Acad. Sci. USA 76, 4350-4354.
von Heijne (1983), Eur. J. Biochem. 133, 17-21.
Wilson & Ingledew (1982), Appl. Environ. Microbiol. 44, 301-307.
Zalkin & Yanowsky (1982), J. Biol. Chem. 257, 1491-1500.

## Claims (Claims for the following Contracting State(s): AT, BE, CH, LI, DE, FR, GB, GR, IT, LU, NL, SE)

1. A yeast cell of the genus Schwanniomyces,
**characterized in that**
said yeast cell contains at least one expression cassette comprising :
a) a first DNA sequence serving as a regulon,
b) optionally a second DNA sequence coding for a signal peptide
c) a third DNA sequence coding for a foreign protein, and
d) optionally a fourth DNA sequence serving as a terminator,
wherein the first, second and fourth DNA sequence are derived from yeast.

2. The yeast cell according to claim 1,
**characterized in that**
said first and/or second and/or fourth DNA sequence are derived from a gene coding for an amylolytic enzyme.

3. The yeast cell according to claim 2,
**characterized in that**
said amylolytic enzyme is an α-amylase or glucoamylase.

4. The yeast cell according to any of claims 1 to 3,
**characterized in that**
said first and/or second and/or fourth DNA sequences are derived from a yeast of a member of the genera Debariomyces, Saccharomycopses, Lipomyces, Pichia, or Saccharomyces, preferably Schwanniomyces.

5. The yeast cell according to claim 4,
**characterized in that**
said yeast is Schwanniomyces occidentalis.

6. The yeast cell according to any of claims 1 to 5,
**characterized in that**
said first DNA sequence is comprised by a 1.8 kb BglII-XhoI-fragment, if derived from Schwanniomyces occidentalis α-amylase gene, or a 1.3 kb BamHI-PvuII fragment if derived from Schwanniomvces occidentalis glucoamylase gene.

7. The yeast cell according to claim 5,
**characterized in that**
said first DNA sequence is comprised by part or all of the DNA sequence corresponding to bases -1 to -540 of the region preceeding the structural gene coding for α-amylase or part or all of the DNA sequence corresponding to bases -1 to -320 of the region preceeding the structural gene coding for glucoamylase.

8. The yeast cell according to any of claims 1 to 5,
**characterized in that**
said first DNA sequence corresponds to one of the regulons regulating expression of:
a) ADH1,ADH2, PDC1, GAL1/10, PGK, GAPDH, wherein the regulon is preferably obtained from Saccharomyces cerevisiae,
b) LAC4, wherein the regulon is preferably obtained from Kluyveromyces lactis, or
c) corresponding regulons, preferably obtained from Schwanniomyces.

9. The yeast cell according to any of claims 1 to 8, **characterized in that** said second DNA sequence is coding for all or part of at least one of the following peptides :

a) MetArgPheSerThrGluGlyPheThrSerLysValValAlaAlaIle
LeuAlaPheSerArgLeuValSerAla;

b)  MetArgPheSerThrGluGlyPheThrSerLysValValAlaAlaIle
LeuAlaPheSerArgLeuValSerAlaGlnProIleIlePheAspMetArg;

c) MetIlePheLeuLysLeuIleLysSerIleValIleGlyLeuGlyLeu
ValSerAla;

d)  MetIlePheLeuLysLeuIleLysSerIleValIleGlyLeuGlyLeu
ValSerAlaIleGlnAlaAlaProAla;

e) MetIlePheLeuLysLeuIleLysSerIleValILeGlyLeuGlyLeu
ValSerAlaIleGlnAlaAlaProAlaSerSerIleGlySerSerAlaSerAla

10. A yeast cell according to claim 9,
**characterized in that**
the DNA sequence coding for said peptide corresponds to part or all of one of the following DNA sequences:

a) ATGAGATTTTCAACTGAAGGATTTACAAGTAAAGTTGTTGCAGCAATT
TTAGCATTCTCAAGATTGGTATCTGCT,

b) ATGAGATTTTCAACTGAAGGATTTACAAGTAAAGTTGTTGCAGCAATTT
TAGCATTCTCAAGATTGGTATCTGCTCAACCGATTATTTTTGACATGAGA;

c) ATGATTTTTCTGAAGCTGATTAAAAGTATAGTAATTGGTTTGGGATTA
GTTAGTGCT;

d)    ATGATTTTTCTGAAGCTGATTAAAAGTATAGTAATTGGTTTGGGATTA
GTTAGTGCTATCCAAGCAGCCCCTGCC;

e) ATGATTTTTCTGAAGCTGATTAAAAGTATAGTAATTGGTTTGGGATTA
GTTAGTGCTATCCAAGCAGCCCCTGCCTCTTCGATTGGATCTAGTGCTTCA
GCA.

11. The yeast cell according to any of claims 1 to 10,
**characterized in that**
said third DNA sequence coding for a foreign protein is a natural or synthetic DNA sequence encoding a cellulase, interleukin, insulin like growth factor, interferon, lymphokine, human growth factor, nerve growth factor, aprotinin, insulin, hirudin, hormones, blood clotting factors, hepatitis B surface or core antigens, viral or bacterial vaccines or human granulocyte/macrophage colony stimulating factor.

12. The yeast cell according to any of claims 1 to 11,
**characterized in that**
said fourth DNA sequence originates from a yeast of a member of the genera Saccharomyces, Pichia, Hansenula, preferably Schwanniomyces.

13. The yeast cell according to claim 12,
**characterized in that**
said fourth DNA sequence corresponds to part or all of the terminator comprised by nucleotides 1537 to 1740 of the Schwanniomyces α-amylase gene or part or all of the terminator comprised by nucleotides 2875 to 3320 of the Schwanniomyces glucoamylase gene.

14. The yeast cell according to any of claims 1 to 13,
**characterized in that**
said first and/or second and/or third and/or fourth DNA sequence has been modified by insertion or deletion or substitution of one or more nucleotides while the biological activity of the DNA sequence is retained or improved.

15. The yeast cell according to any of claims 1 to 14,
**characterized in that**
said expression cassette is carried by a circular or linear vector, which optionally comprises one or more selective marker genes.

16. The yeast cell according to claim 15,
**characterized in that**
said vector comprises any of the following selective marker genes : TRP5, LEU2, ADE1, ADE2, HIS3, HIS4, URA3, LYS2, preferably obtained from Saccharomyces, Hansenula Pichia or Schwanniomyces, AMY1 or GAM1, preferably obtained from Schwanniomyces.

17. The yeast cell according to any of claims 15 and 16,
**characterized in that**
said vector further comprises a DNA sequence capable of controlling autonomous replication and stable maintenance of the vector in the host organism.

18. The yeast cell according to claim 17
**characterized in that**
said DNA sequence is derived from the genome of a member of the genera Saccharomyces, Pichia, Hansenula or Kluyveromyces, preferably Schwanniomyces.

19. A yeast cell according to any of claims 17 or 18,
**characterized in that**
said DNA sequence is selected from the autonomously replicating sequences ARS, preferably an ARS derived from the respective host organism.

20. The yeast cell according to claim 19,
**characterized in that**
said DNA sequence is SwARS1 or SwARS2, capable of controlling autonomous replication and stable maintenance of the vector in Schwanniomyces, wherein SwARS1 is comprised on a ClaI-BamHI fragment adjacent to the EcoRI fragment shown in Fig. 2a, and SwARS2 is comprised on an EcoRI-BglII fragment contained in the EcoRI fragment shown in Fig. 2a.

21. The yeast cell according to any of claims 17 to 20,
**characterized in that**
said DNA sequence has been modified by deletion, insertion or substitution of one or more nucleotides, while retaining or improving its biological function.

22. The yeast cell according to any of claims 1 to 21,
**characterized in that**
said expression cassette is integrated into the genome, optionally in multiple copies, preferably via homologous recombination.

23. A process for the production of polypeptide, wherein a yeast host organism is cultivated under suitable conditions and the polypeptide is recovered in a manner known per se,
**characterized in that**
said yeast host organism is a yeast cell according to any of claims 1 to 22.

24. The process according to claim 23
**characterized in that**
yeast cells of the species Schwanniomyces occidentalis are used as a host organism and are preferably cultivated on starch dextrin, maltose and/or plant biomass.

25. The process according to any of claims 23 or 24,
**characterized in that**
yeast cells of the genus Schwanniomyces are used for the production of single cell protein.

26. The process according to any of claims 23 to 25,
**characterized in that**
said polypeptide is amylase or glucoamylase.

27. Use of a yeast cell according to any of claim 1 to 22 for transformation and expression of foreign genes.

## Claims (Claims for the following Contracting State(s): ES)

1. A process for preparing a yeast cell capable of expressing foreign protein,
**characterized in that**
at least one expression cassette comprising:
a) a first DNA sequence serving as a regulon,
b) optionally a second DNA sequence coding for a signal peptide
c) a third DNA sequence coding for a foreign protein, and
d) optionally a fourth DNA sequence serving as a terminator,
is inserted into a yeast cell of the genus Schwanniomyces, wherein the first, second and fourth DNA sequence are derived from yeast.

2. The process according to claim 1,
**characterized in that**
said first and/or second and/or fourth DNA sequence are derived from a gene coding for a amylolytic enzyme.

3. The process according to claim 2,
**characterized in that**
said amylolytic enzyme is an α-amylase or glucoamylase.

4. The process according to any of claims 1 to 3,
**characterized in that**
said first and/or second and/or fourth DNA sequences are derived from a yeast of a member of the genera Debariomyces, Saccharomycopses, Lipomyces, Pichia, or Saccharomyces, preferably Schwanniomyces.

5. The process according to claim 4,
**characterized in that**
said yeast is Schwanniomyces occidentalis.

6. The process according to any of claims 1 to 5,
**characterized in that**
said first DNA sequence is comprised by a 1.8 kb BglII-XhoI-fragment, if derived from Schwanniomyces occidentalis α-amylase gene, or a 1.3 kb BamHI-PvuII fragment if derived from Schwanniomyces occidentalis glucoamylase gene.

7. The process according to claim 5,
**characterized in that**
said first DNA sequence is comprised by part or all of the DNA sequence corresponding to bases -1 to -540 of the region preceeding the structural gene coding for α-amylase or part or all of the DNA sequence corresponding to bases -1 to -320 of the region preceeding the structural gene coding for glucoamylase.

8. The process according to any of claims 1 to 5,
**characterized in that**
said first DNA sequence corresponds to one of the regulons regulating expression of:
a) ADH1,ADH2, PDC1, GAL1/10, PGK, GAPDH, wherein the regulon is preferably obtained from Saccharomyces cerevisiae,
b) LAC4, wherein the regulon is preferably obtained from Kluyveromyces lactis, or
c) corresponding regulons, preferably obtained from Schwanniomyces.

9. The process according to any of claims 1 to 8,
**characterized in that**
said second DNA sequence is coding for all or part of at least one of the following peptides :

a) MetArgPheSerThrGluGlyPheThrSerLysValValAlaAlaIle
LeuAlaPheSerArgLeuValSerAla;

b) MetArgPheSerThrGluGlyPheThrSerLysValValAlaAlaIle
LeuAlaPheSerArgLeuValSerAlaGlnProIleIlePheAspMetArg;

c) MetIlePheLeuLysLeuIleLysSerIleValIleGlyLeuGlyLeu
ValSerAla;

d) MetIlePheLeuLysLeuIleLysSerIleValIleGlyLeuGlyLeu
ValSerAlaIleGlnAlaAlaProAla;

e) MetIlePheLeuLysLeuIleLysSerIleValILeGlyLeuGlyLeu
ValSerAlaIleGlnAlaAlaProAlaSerSerIleGlySerSerAlaSerAla

10. A process according to claim 9,
**characterized in that**
the DNA sequence coding for said peptide corresponds to part or all of one of the following DNA sequences:

a)  ATGAGATTTTCAACTGAAGGATTTACAAGTAAAGTTGTTGCAGCAATT
    TTAGCATTCTCAAGATTGGTATCTGCT,

b)    ATGAGATTTTCAACTGAAGGATTTACAAGTAAAGTTGTTGCAGCAATTT
       TAGCATTCTCAAGATTGGTATCTGCTCAACCGATTATTTTTGACATGAGA;

c)  ATGATTTTTCTGAAGCTGATTAAAAGTATAGTAATTGGTTTGGGATTA
    GTTAGTGCT;

d)  ATGATTTTTCTGAAGCTGATTAAAAGTATAGTAATTGGTTTGGGATTA
GTTAGTGCTATCCAAGCAGCCCCTGCC;

e) ATGATTTTTCTGAAGCTGATTAAAAGTATAGTAATTGGTTTGGATTA
GTTAGTGCTATCCAAGCAGCCCCTGCCTCTTCGATTGGATCTAGTGCTTCA
GCA.

11. The process according to any of claims 1 to 10,
**characterized in that**
said third DNA sequence coding for a foreign protein is a natural or synthetic DNA sequence encoding a cellulase, interleukin , insulin like growth factor, interferon, lymphokine, human growth factor, nerve growth factor, aprotinin, insulin, hirudin, hormones, blood clotting factors, hepatitis B surface or core antigens, viral or bacterial vaccines or human granulocyte/macrophage colony stimulating factor.

12. The process according to any of claims 1 to 11,
**characterized in that**
said fourth DNA sequence originates from a yeast of a member of the genera Saccharomyces, Pichia, Hansenula, preferably Schwanniomyces.

13. The process according to claim 12,
**characterized in that**
said fourth DNA sequence corresponds to part or all of the terminator comprised by nucleotides 1537 to 1740 of the Schwanniomyces α-amylase gene or part or all of the terminator comprised by nucleotides 2875 to 3320 of the Schwanniomyces glucoamylase gene.

14. The process according to any of claims 1 to 13,
**characterized in that**
said first and/or second and/or third and/or fourth DNA sequence has been modified by insertion or deletion or substitution of one or more nucleotides while the biological activity of the DNA sequence is retained or improved.

15. The process according to any of claims 1 to 14,
**characterized in that**
said expression cassette is carried by a circular or linear vector, which optionally comprises one or more selective marker genes.

16. The process according to claim 15,
**characterized in that**
said vector comprises any of the following selective marker genes : TRP5, LEU2, ADE1, ADE2, HIS3, HIS4, URA3, LYS2, preferably obtained from Saccharomyces, Hansenula Pichia or Schwanniomyces, AMY1 or GAM1, preferably obtained from Schwanniomyces.

17. The process according to any of claims 15 and 16,
**characterized in that**
said vector further comprises a DNA sequence capable of controlling autonomous replication and stable maintenance of the vector in the host organism.

18. The process according to claim 17
**characterized in that**
said DNA sequence is derived from the genome of a member of the genera Saccharomyces, Pichia, Hansenula or Kluyveromyces, preferably Schwanniomyces.

19. A process according to any of claims 17 or 18,
**characterized in that**
said DNA sequence is selected from the autonomously replicating sequences ARS, preferably an ARS derived from the respective host organism.

20. The process according to claim 19,
**characterized in that**
said DNA sequence is SwARS1 or SwARS2, capable of controlling autonomous replication and stable maintenance of the vector in Schwanniomyces, wherein SwARS1 is comprised on a ClaI-BamHI fragment adjacent to the EcoRI fragment shown in Fig. 2a, and SwARS2 is comprised on an EcoRI-BglII fragment contained in the EcoRI fragment shown in Fig 2a.

21. The process according to any of claims 17 to 20,
**characterized in that**
said DNA sequence has been modified by deletion, insertion or substitution of one or more nucleotides, while retaining or improving its biological function.

22. The process according to any of claims 1 to 21,
**characterized in that**
said expression cassette is integrated into the genome, optionally in multiple copies, preferably via homologous recombination.

23. A process for the production of polypeptide, wherein a yeast host organism is cultivated under suitable conditions and the polypeptide is recovered in a manner known per se,
**characterized in that**
said yeast host organism is a yeast cell according to any of claims 1 to 22.

24. The process according to claim 23
**characterized in that**
yeast cells of the species Schwanniomyces occidentalis are used as a host organism and are preferably cultivated on starch dextrin, maltose and/or plant biomass.

25. The process according to any of claims 23 or 24,
**characterized in that**
yeast cells of the genus Schwanniomyces are used for the production of single cell protein.

26. The process according to any of claims 23 to 25,
**characterized in that**
said polypeptide is amylase or glucoamylase.

27. Use of a yeast cell according to any of claim 1 to 22 for transformation and expression of foreign genes.

## Patentansprüche (Patentansprüche für folgende(n) Vertragsstaat(en): AT, BE, CH, LI, DE, FR, GB, GR, IT, LU, NL, SE)

1. Hefezelle der Gattung Schwanniomyces, dadurch gekennzeichnet, daß die Hefezelle mindestens eine Expressionskassette enthält, umfassend:
a) eine erste DNA-Sequenz, die als Regulon dient,
b) gegebenenfalls eine zweite DNA-Sequenz, die für ein Signalpeptid kodiert,
c) eine dritte DNA-Sequenz, die für ein Fremdprotein kodiert, und
d) gegebenenfalls eine vierte DNA-Sequenz, die als ein Terminator dient,
wobei die erste, zweite und vierte DNA-Sequenz von Hefe abgeleitet sind.

2. Hefezelle nach Anspruch 1, dadurch gekennzeichnet, daß die erste und/oder zweite und/oder vierte DNA-Sequenz von einem für ein amylolytisches Enzym kodierenden Gen abgeleitet sind.

3. Hefezelle nach Anspruch 2, dadurch gekennzeichnet, daß das amylolytische Enzym eine α-Amylase oder Glucoamylase ist.

4. Hefezelle nach einem der Ansprüche 1 bis 3, dadurch gekennzeichnet, daß die ersten und/oder zweiten und/oder vierten DNA-Sequenzen von einer Hefe eines Angehörigen der Gattungen Debariomyces, Saccharomycopses, Lipomyces, Pichia oder Saccharomyces, bevorzugt Schwanniomyces, abgeleitet sind.

5. Hefezelle nach Anspruch 4, dadurch gekennzeichnet, daß die Hefe Schwanniomyces occidentalis ist.

6. Hefezelle nach einem der Ansprüche 1 bis 5, dadurch gekennzeichnet, daß die erste DNA-Sequenz von einem 1,8 kb BglII-XhoI-Fragment umfaßt ist, wenn sie vom Schwanniomyces occidentalis-α-Amylasegen abgeleitet ist, oder von einem 1,3 kb BamHI-PvuII-Fragment, wenn sie von einem Schwanniomyces occidentalis-Glucoamylasegen abgeleitet ist.

7. Hefezelle nach Anspruch 5, dadurch gekennzeichnet, daß die erste DNA-Sequenz von einem Teil oder der gesamten DNA-Sequenz umfaßt ist, die den Basen -1 bis -540 des Bereiches entspricht, der dem für α-Amylase kodierenden Strukturgen vorangeht, oder einem Teil oder der Gesamtheit der DNA-Sequenz, die den Basen -1 bis -320 des Bereiches entspricht, der dem für Glucoamylase kodierenden Strukturgen vorangeht.

8. Hefezelle nach einem der Ansprüche 1 bis 5, dadurch gekennzeichnet, daß die erste DNA-Sequenz einem der Regulons entspricht, die die Expression regulieren von
a) ADH1, ADH2, PDC1, GAL1/10, PGK, GAPDH, wobei das Regulon bevorzugt von Saccharomyces cerevisiae erhalten ist,
b) LAC4, wobei das Regulon bevorzugt von Kluyveromvces lactis erhalten ist, oder
c) entsprechenden Regulons, bevorzugt aus Schwanniomyces erhalten.

9. Hefezelle nach einem der Ansprüche 1 bis 8, dadurch gekennzeichnet, daß die zweite DNA-Sequenz für die Gesamtheit oder einen Teil mindestens eines der folgenden Peptide kodiert:

a)  MetArgPheSerThrGluGlyPheThrSerLysValValAlaAlaIle
LeuAlaPheSerArgLeuValSerAla;

b) MetArgPheSerThrGluGlyPheThrSerLysValValAlaAlaIle
LeuAlaPheSerArgLeuValSerAlaGlnProIleIlePheAspMetArg;

c) MetIlePheLeuLysLeuIleLysSerIleValIleGlyLeuGlyLeu
ValSerAla;

d)  MetIlePheLeuLysLeuIleLysSerIleValIleGlyLeuGlyLeu
ValSerAlaIleGlnAlaAlaProAla;

e)  MetIlePheLeuLysLeuIleLysSerIleValILeGlyLeuGlyLeu
ValSerAlaIleGlnAlaAlaProAlaSerSerIleGlySerSerAlaSerAla

10. Hefezelle nach Anspruch 9, dadurch gekennzeichnet, daß die für das Peptid kodierende DNA-Sequenz einem Teil oder der Gesamtheit einer der folgenden DNA-Sequenzen entspricht:

a) ATGAGATTTTCAACTGAAGGATTTACAAGTAAAGTTGTTGCAGCAATT
TTAGCATTCTCAAGATTGGTATCTGCT,

b)  ATGAGATTTTCAACTGAAGGATTTACAAGTAAAGTTGTTGCAGCAATTT
    TAGCATTCTCAAGATTGGTATCTGCTCAACCGATTATTTTTGACATGAGA;

c) ATGATTTTTCTGAAGCTGATTAAAAGTATAGTAATTGGTTTGGATTA
GTTAGTGCT;

d)    ATGATTTTTCTGAAGCTGATTAAAAGTATAGTAATTGGTTTGGATTA
      GTTAGTGCTATCCAAGCAGCCCCTGCC;

e)	ATGATTTTTCTGAAGCTGATTAAAAGTATAGTAATTGGTTTGGATTA
GTTAGTGCTATCCAAGCAGCCCCTGCCTCTTCGATTGGATCTAGTGCTTCA
GCA.

11. Hefezelle nach einem der Ansprüche 1 bis 10, dadurch gekennzeichnet, daß die dritte DNA-Sequenz, die für ein Fremdprotein kodiert, eine natürliche oder synthetische DNA-Sequenz ist, die für eine Zellulase, Interleukin, insulinähnlichen Wachstumsfaktor, Interferon, Lymphokin, menschlichen Wachstumsfaktor, Nervenwachstumsfaktor, Aprotinin, Insulin, Hirudin, Hormone, Blutgerinnungsfaktoren, Hepatitis-B-Oberflächen- oder -Kernantigene, virale oder bakterielle Impfstoffe oder humanen Granulocyten/Makrophagen-Kolonie-stimulierenden Faktor kodiert.

12. Hefezelle nach einem der Ansprüche 1 bis 11, dadurch gekennzeichnet, daß die vierte DNA-Sequenz von einer Hefe eines Angehörigen der Gattungen Saccharomyces, Pichia, Hansenula, bevorzugt Schwanniomyces, stammt.

13. Hefezelle nach Anspruch 12, dadurch gekennzeichnet, daß die vierte DNA-Sequenz einem Teil oder der Gesamtheit des Terminators entspricht, der von den Nukleotiden 1537 bis 1740 des Schwanniomyces-α-Amylasegenes umfaßt ist, oder einem Teil oder der Gesamtheit des Terminators, der von den Nukleotiden 2875 bis 3320 des Schwanniomyces-Glucoamylasegenes umfaßt ist.

14. Hefezelle nach einem der Ansprüche 1 bis 13, dadurch gekennzeichnet, daß die erste und/oder zweite und/oder dritte und/oder vierte DNA-Sequenz durch Insertion oder Deletion oder Substitution eines oder mehrerer Nukleotide modifiziert worden ist, während die biologische Aktivität der DNA-Sequenz beibehalten oder verbessert wird.

15. Hefezelle nach einem der Ansprüche 1 bis 14, dadurch gekennzeichnet, daß die Expressionskassette von einem zirkulären oder linearen Vektor getragen wird, der gegebenenfalls ein oder mehrere selektive Markergene umfaßt.

16. Hefezelle nach Anspruch 15, dadurch gekennzeichnet, daß der Vektor eines der folgenden selektiven Markergene umfaßt:
TRP5, LEU2, ADE1, ADE2, HIS3, HIS4, URA3, LYS2, bevorzugt erhalten von Saccharomyces, Hansenula, Pichia oder Schwanniomyces, AMY1 oder GAM1, bevorzugt erhalten von Schwanniomyces.

17. Hefezelle nach einem der Ansprüche 15 und 16, dadurch gekennzeichnet, daß der Vektor weiter eine DNA-Sequenz umfaßt, die die autonome Replikation und stabile Beibehaltung des Vektors in dem Wirtsorganismus steuern kann.

18. Hefezelle nach Anspruch 17, dadurch gekennzeichnet, daß die DNA-Sequenz von dem Genom eines Angehörigen der Gattungen Saccharomyces, Pichia, Hansenula oder Kluyveromyces abgeleitet ist, bevorzugt Schwanniomyces.

19. Hefezelle nach einem der Ansprüche 17 oder 18, dadurch gekennzeichnet, daß die DNA-Sequenz aus den autonom replizierenden Sequenzen ARS, bevorzugt einer ARS, die von dem jeweiligen Wirtsorganismus abgeleitet ist, ausgewählt ist.

20. Hefezelle nach Anspruch 19, dadurch gekennzeichnet, daß die DNA-Sequenz SwARS1 oder SwARS2 ist, die die autonome Replikation und stabile Beibehaltung des Vektors in Schwanniomyces steuern können, wobei SwARS1 von einem ClaI-BamHI-Fragment umfaßt ist, das dem in Figur 2a gezeigten EcoRI-Fragment benachbart ist, und SwARS2 von einem EcoRI- BglII-Fragment umfaßt ist, das in dem in Figur 2a gezeigten EcoRI-Fragment enthalten ist.

21. Hefezelle nach einem der Ansprüche 17 bis 20, dadurch gekennzeichnet, daß die DNA-Sequenz durch Deletion, Insertion oder Substitution eines oder mehrerer Nukleotide modifiziert worden ist, während ihre biologische Funktion beibehalten oder verbessert ist.

22. Hefezelle nach einem der Ansprüche 1 bis 21, dadurch gekennzeichnet, daß die Expressionskassette in das Genom integriert wird, gegebenenfalls in mehreren Kopien, bevorzugt über homologe Rekombination.

23. Verfahren zum Erzeugen von Polypeptid, wobei ein Hefewirtsorganismus unter geeigneten Bedingungen kultiviert und das Polypeptid in an sich bekannter Weise gewonnen wird, dadurch gekennzeichnet, daß der Hefeorganismus eine Hefezelle gemäß einem der Ansprüche 1 bis 22 ist.

24. Verfahren nach Anspruch 23, dadurch gekennzeichnet, daß Hefezellen der Art Schwanniomyces occidentalis als ein Wirtsorganismus verwendet werden und bevorzugt auf Stärke, Dextrin, Maltose und/oder Pflanzenbiomasse kultiviert werden.

25. Verfahren nach einem der Ansprüche 23 oder 24, dadurch gekennzeichnet, daß Hefezellen der Gattung Schwanniomyces für die Erzeugung von Einzelzellprotein verwendet werden.

26. Verfahren nach einem der Ansprüche 23 bis 25, dadurch gekennzeichnet, daß das Polypeptid Amylase oder Glycoamylase ist.

27. Verwendung einer Hefezelle nach einem der Ansprüche 1 bis 22 zur Transformation und Expression von Fremdgenen.

## Patentansprüche (Patentansprüche für folgende(n) Vertragsstaat(en): ES)

1. Verfahren zum Herstellen einer Hefezelle, die Fremdprotein exprimieren kann, dadurch gekennzeichnet, daß mindestens eine Expressionskassette in eine Hefezelle der Gattung Schwanniomyces insertiert wird, wobei die Expressionskassette umfaßt:
a) eine erste DNA-Sequenz, die als Regulon dient,
b) gegebenenfalls eine zweite DNA-Sequenz, die für ein Signalpeptid kodiert,
c) eine dritte DNA-Sequenz, die für ein Fremdprotein kodiert, und
d) gegebenenfalls eine vierte DNA-Sequenz, die als Terminator dient,
wobei die erste, zweite und vierte DNA-Sequenz von Hefe abgeleitet sind.

2. Verfahren nach Anspruch 1, dadurch gekennzeichnet, daß die erste und/oder zweite und/oder vierte DNA-Sequenz von einem für ein amylolytisches Enzym kodierenden Gen abgeleitet sind.

3. Verfahren nach Anspruch 2, dadurch gekennzeichnet, daß das amylolytische Enzym eine α-Amylase oder Glucoamylase ist.

4. Verfahren nach einem der Ansprüche 1 bis 3, dadurch gekennzeichnet, daß die ersten und/oder zweiten und/oder vierten DNA-Sequenzen von einer Hefe eines Angehörigen der Gattungen Debariomyces, Saccharomycopses, Lipomyces, Pichia oder Saccharomyces, bevorzugt Schwanniomyces, abgeleitet sind.

5. Verfahren nach Anspruch 4, dadurch gekennzeichnet, daß die Hefe Schwanniomyces occidentalis ist.

6. Verfahren nach einem der Ansprüche 1 bis 5, dadurch gekennzeichnet, daß die erste DNA-Sequenz von einem 1,8 kb BglII-XhoI-Fragment umfaßt ist, wenn sie vom Schwanniomyces occidentalis-α-Amylasegen abgeleitet ist, oder von einem 1,3 kb BamHI-PvuII-Fragment umfaßt ist, wenn sie vom Schwanniomyces occidentalis-Glucoamylasegen abgeleitet ist.

7. Verfahren nach Anspruch 5, dadurch gekennzeichnet, daß die erste DNA-Sequenz von einem Teil oder der Gesamtheit der DNA-Sequenz umfaßt ist, die den Basen -1 bis -540 des Bereiches entspricht, der dem für α-Amylase kodierenden Strukturgen vorangeht, oder einem Teil oder der Gesamtheit der DNA-Sequenz, die den Basen -1 bis -320 des Bereichs entspricht, der dem für Glucoamylase kodierenden Strukturgen vorangeht.

8. Verfahren nach einem der Ansprüche 1 bis 5, dadurch gekennzeichnet, daß die erste DNA-Sequenz einem der Regulons entspricht, die die Expression regulieren von
a) ADH1, ADH2, PDC1, GAL1/10, PGK, GAPDH, wobei das Regulon bevorzugt von Saccharomyces cerevisiae erhalten ist,
b) LAC4, wobei das Regulon bevorzugt von Klyveromyces lactis erhalten ist, oder
c) korrespondierenden Regulons, bevorzugt von Schwanniomyces erhalten.

9. Verfahren nach einem der Ansprüche 1 bis 8, dadurch gekennzeichnet, daß die zweite DNA-Sequenz für die Gesamtheit oder einen Teil mindestens eines der folgenden Peptide kodiert:

a) MetArgPheSerThrGluGlyPheThrSerLysValValAlaAlaIle
LeuAlaPheSerArgLeuValSerAla;

b) MetArgPheSerThrGluGlyPheThrSerLysValValAlaAlaIle
LeuAlaPheSerArgLeuValSerAlaGlnProIleIlePheAspMetArg;

c) MetIlePheLeuLysLeuIleLysSerIleValIleGlyLeuGlyLeu
ValSerAla;

d) MetIlePheLeuLysLeuIleLysSerIleValIleGlyLeuGlyLeu
ValSerAlaIleGlnAlaAlaProAla;

e) MetIlePheLeuLysLeuIleLysSerIleValILeGlyLeuGlyLeu
ValSerAlaIleGlnAlaAlaProAlaSerSerIleGlySerSerAlaSerAla

10. Verfahren nach Anspruch 9, dadurch gekennzeichnet, daß die für das Peptid kodierende DNA-Sequenz einem Teil oder der Gesamtheit einer der folgenden DNA-Sequenzen entspricht:

a) ATGAGATTTTCAACTGAAGGATTTACAAGTAAAGTTGTTGCAGCAATT
TTAGCATTCTCAAGATTGGTATCTGCT,

b) ATGAGATTTTCAACTGAAGGATTTACAAGTAAAGTTGTTGCAGCAATTT
TAGCATTCTCAAGATTGGTATCTGCTCAACCGATTATTTTTGACATGAGA;

c) ATGATTTTTCTGAAGCTGATTAAAAGTATAGTAATTGGTTTGGATTA
GTTAGTGCT;

d)  ATGATTTTTCTGAAGCTGATTAAAAGTATAGTAATTGGTTTGGATTA
GTTAGTGCTATCCAAGCAGCCCCTGCC;

e) ATGATTTTTCTGAAGCTGATTAAAAGTATAGTAATTGGTTTGGATTA
GTTAGTGCTATCCAAGCAGCCCCTGCCTCTTCGATTGGATCTAGTGCTTCA
GCA.

11. Verfahren nach einem der Ansprüche 1 bis 10, dadurch gekennzeichnet, daß die dritte DNA-Sequenz, die für ein Fremdprotein kodiert, eine natürliche oder synthetische DNA-Sequenz ist, die für eine Zellulase, Interleukin, insulinähnlichen Wachstumsfaktor, Interferon, Lymphokin, menschlichen Wachstumsfaktor, Nervenwachstumsfaktor, Aprotinin, Insulin, Hirudin, Hormone, Blutgerinnungsfaktoren, Hepatitis-B-Oberflächen- oder -Kernantigene, virale oder bakterielle Impfstoffe oder humanen Granulocyten/Makrophagen-Kolonie-stimulierenden Faktor kodiert.

12. Verfahren nach einem der Ansprüche 1 bis 11, dadurch gekennzeichnet, daß die vierte DNA-Sequenz von einer Hefe eines Angehörigen der Gattungen Saccharomyces, Pichia, Hansenula, bevorzugt Schwanniomyces, stammt.

13. Verfahren nach Anspruch 12, dadurch gekennzeichnet, daß die vierte DNA-Sequenz einem Teil oder der Gesamtheit des Terminators entspricht, der von den Nukleotiden 1537 bis 1740 des Schwanniomyces-α-Amylasegenes umfaßt ist, oder einem Teil oder der Gesamtheit des Terminators, der von den Nukleotiden 2875 bis 3320 des Schwanniomyces-Glucoamylasegenes umfaßt ist.

14. Verfahren nach einem der Ansprüche 1 bis 13, dadurch gekennzeichnet, daß die erste und/oder zweite und/oder dritte und/oder vierte DNA-Sequenz durch Insertion oder Deletion oder Substitution eines oder mehrerer Nukleotide modifiziert worden ist, während die biologische Aktivität der DNA-Sequenz beibehalten oder verbessert wird.

15. Verfahren nach einem der Ansprüche 1 bis 14, dadurch gekennzeichnet, daß die Expressionskassette von einem zirkulären oder linearen Vektor getragen wird, der gegebenenfalls ein oder mehrere selektive Markergene umfaßt.

16. Verfahren nach Anspruch 15, dadurch gekennzeichnet, daß der Vektor eines der folgenden selektiven Markergene umfaßt:
TRP5, LEU2, ADE1, ADE2, HIS3, HIS4, URA3, LYS2, bevorzugt erhalten von Saccharomyces, Hansenula, Pichia oder Schwanniomyces, AMY1 oder GAM1, bevorzugt erhalten von Schwanniomyces.

17. Verfahren nach einem der Ansprüche 15 und 16, dadurch gekennzeichnet, daß der Vektor weiter eine DNA-Sequenz umfaßt, die die autonome Replikation und stabile Beibehaltung des Vektors in dem Wirtsorganismus steuern kann.

18. Verfahren nach Anspruch 17, dadurch gekennzeichnet, daß die DNA-Sequenz von dem Genom eines Angehörigen der Gattungen Saccharomyces, Pichia, Hansenula oder Kluyveromyces abgeleitet ist, bevorzugt Schwanniomyces.

19. Verfahren nach einem der Ansprüche 17 oder 18, dadurch gekennzeichnet, daß die DNA-Sequenz aus den autonom replizierenden Sequenzen ARS, bevorzugt einer ARS, die von dem jeweiligen Wirtsorganismus abgeleitet ist, ausgewählt wird.

20. Verfahren nach Anspruch 19, dadurch gekennzeichnet, daß die DNA-Sequenz SwARS1 oder SwARS2 ist, die die autonome Replikation und stabile Beibehaltung des Vektors in Schwanniomyces steuern können, wobei SwARS1 von einem ClaI-BamHI-Fragment umfaßt ist, das dem in Figur 2a gezeigten EcoRI-Fragment benachbart ist, und SwARS2 von einem EcoRI-BglII-Fragment umfaßt ist, das in dem in Figur 2a gezeigten EcoRI-Fragment enthalten ist.

21. Verfahren nach einem der Ansprüche 17 bis 20, dadurch gekennzeichnet, daß die DNA-Sequenz durch Deletion, Insertion oder Substitution eines oder mehrerer Nukleotide modifiziert worden ist, während ihre biologische Funktion beibehalten oder verbessert wird.

22. Verfahren nach einem der Ansprüche 1 bis 21, dadurch gekennzeichnet, daß die Expressionskassette in das Genom integriert ist, gegebenenfalls in mehrfachen Kopien, bevorzugt über homologe Rekombination.

23. Verfahren zum Erzeugen eines Polypeptides, wobei ein Hefewirtsorganismus unter geeigneten Bedingungen kultiviert und das Polypeptid in an sich bekannter Weise gewonnen wird, dadurch gekennzeichnet, daß der Hefewirtsorganismus eine Hefezelle gemäß einem der Ansprüche 1 bis 22 ist.

24. Verfahren nach Anspruch 23, dadurch gekennzeichnet, daß Hefezellen der Art Schwanniomyces occidentalis als ein Wirtsorganismus verwendet werden und bevorzugt auf Stärke, Dextrin, Maltose und/oder Pflanzenbiomasse kultiviert werden.

25. Verfahren nach einem der Ansprüche 23 oder 24, dadurch gekennzeichnet, daß Hefezellen der Gattung Schwanniomyces für die Erzeugung von Einzelzellprotein verwendet werden.

26. Verfahren nach einem der Ansprüche 23 bis 25, dadurch gekennzeichnet, daß das Polypeptid Amylase oder Glycoamylase ist.

27. Verwendung einer Hefezelle gemäß einem der Ansprüche 1 bis 22 zur Transformation und Expression von Fremdgenen.

## Revendications (Revendications pour l'(les) Etat(s) contractant(s) suivant(s): AT, BE, CH, LI, DE, FR, GB, GR, IT, LU, NL, SE)

1. Cellule de levure du genre *Schwanniomyces,* **caractérisée en ce que** ladite cellule de levure contient au moins une cassette d'expression comprenant :
a) une première séquence d'ADN-servant de régulon,
b) facultativement, une deuxième séquence d'ADN codant pour un peptide signal
c) une troisième séquence d'ADN codant pour une protéine étrangère, et
d) facultativement, une quatrième séquence d'ADN servant de terminateur,
les première, deuxième et quatrième séquences d'ADN étant obtenues à partir d'une levure.

2. Cellule de levure selon la revendication 1, **caractérisée en ce que** lesdites première et/ou deuxième et/ou quatrième séquences d'ADN sont obtenues à partir d'un gène codant pour une enzyme amylolytique.

3. Cellule de levure selon la revendication 2, **caractérisée en ce que** ladite enzyme amylolytique est une α-amylase ou la glucoamylase.

4. Cellule de levure selon l'une quelconque des revendications 1 à 3, **caractérisée en ce que** lesdites première et/ou deuxième et/ou quatrième séquences d'ADN sont obtenues à partir d'une levure d'un élément des genres Debariomyces, *Saccharomycopses, Lipomyces, Pichia* ou *Saccharomyces,* de préférence *Schwanniomyces.*

5. Cellule de levure selon la revendication 4, **caractérisée en ce que** ladite levure est *Schwanniomyces occidentalis.*

6. Cellule de levure selon l'une quelconque des revendications 1 à 5, **caractérisée en ce que** ladite première séquence d'ADN est constituée d'un fragment *Bgl*II*-Xho*I de 1,8 kb, si elle est obtenue à partir du gène de la α-amylase de *Schwanniomyces occidentalis,* ou d'un fragment *Bam*HI-*Pvu*II de 1,3 kb si elle est obtenue à partir du gène de la glucoamylase de *Schwanniomyces occidentalis.*

7. Cellule de levure selon la revendication 5, **caractérisée en ce que** ladite première séquence d'ADN est constituée en partie ou en totalité dela séquence d'ADN correspondant aux bases -1 à -540 de la région précédant le gène structural codant pour la α-amylase, ou en partie ou en totalité de la séquence d'ADN correspondant aux bases -1 à -320 de la région précédant le gène structural codant pour la glucoamylase.

8. Cellule de levure selon l'une quelconque des revendications 1 à 5, **caractérisée en ce que** ladite première séquence d'ADN correspond à l'un des régulons régulant l'expression :
a) de ADH1, ADH2, PDC1, GAL1/10, PGK, GAPDH, le régulon étant de préférence obtenu à partir de *Saccharomyces cerevisiae,*
b) de LAC4, le régulon étant de préférence obtenu à partir de *Kluyveromyces lactis,* ou
c) de régulons correspondants, obtenus de préférence à partir de *Schwanniomyces.*

9. Cellule de levure selon l'une quelconque des revendications 1 à 8, **caractérisée en ce que** ladite deuxième séquence d'ADN code pour tout ou partie d'au moins l'un des peptides suivants :

a) MetArgPheSerThrGluGlyPheThrSerLysValValAlaAlaIle
LeuAlaPheSerArgLeuValSerAla;

b)  MetArgPheSerThrGluGlyPheThrSerLysValValAlaAlaIle
LeuAlaPheSerArgLeuValSerAlaGlnProIleIlePheAspMetArg;

c) MetIlePheLeuLysLeuIleLysSerIleValIleGlyLeuGlyLeu
ValSerAla;

d)  MetIlePheLeuLysLeuIleLysSerIleValIleGlyLeuGlyLeu
ValSerAlaIleGlnAlaAlaProAla;

e)  MetIlePheLeuLysLeuIleLysSerIleValILeGlyLeuGlyLeu
ValSerAlaIleGlnAlaAlaProAlaSerSerIleGlySerSerAlaSerAla

10. Cellule de levure selon la revendication 9, **caractérisée en ce que** la séquence d'ADN codant pour ledit peptide correspond à une partie ou à la totalité des séquences d'ADN suivantes :

a) ATGAGATTTTCAACTGAAGGATTTACAAGTAAAGTTGTTGCAGCAATT
TTAGCATTCTCAAGATTGGTATCTGCT,

b) ATGAGATTTTCAACTGAAGGATTTACAAGTAAAGTTGTTGCAGCAATTT
TAGCATTCTCAAGATTGGTATCTGCTCAACCGATTATTTTTGACATGAGA;

c) ATGATTTTTCTGAAGCTGATTAAAAGTATAGTAATTGGTTTGGGATTA
GTTAGTGCT;

d)    ATGATTTTTCTGAAGCTGATTAAAAGTATAGTAATTGGTTTGGGATTA
       GTTAGTGCTATCCAAGCAGCCCCTGCC;

e) ATGATTTTTCTGAAGCTGATTAAAAGTATAGTAATTGGTTTGGATTA
GTTAGTGCTATCCAAGCAGCCCCTGCCTCTTCGATTGGATCTAGTGCTTCA
GCA.

11. Cellule de levure selon l'une quelconque des revendications 1 à 10, **caractérisée en ce que** ladite troisième séquence d'ADN codant pour une protéine étrangère est une séquence d'ADN naturelle ou synthétique codant pour une cellulase, une interleukine, un facteur de croissance semblable à l'insuline, un interféron, la lymphokine, un facteur de croissance humain, un facteur de croissance des nerfs, l'aprotinine, l'insuline, l'hirudine, les hormones, les facteurs de coagulation du sang, les antigènes de surface ou de coeur de l'hépatite B, les vaccins viraux ou bactériens, ou le facteur stimulant une colonie de granulocytes/macrophages humain.

12. Cellule de levure selon l'une quelconque des revendications 1 à 11, **caractérisée en ce que** ladite quatrième séquence d'ADN provient d'une levure d'un élément des genres *Saccharomyces, Pichia, Hansenula,* de préférence *Schwanniomyces.*

13. Cellule de levure selon la revendication 12, **caractérisée en ce que** ladite quatrième séquence d'ADN correspond à une partie ou à la totalité du terminateur constitué des nucléotides 1537 à 1740 du gène de la α-amylase de *Schwanniomyces, ou* à une partie ou à la totalité du terminateur constitué des nucléotides 2875 à 3320 du gène de la glucoamylase de *Schwanniomyces.*

14. Cellule de levure selon l'une quelconque des revendications 1 à 13, **caractérisée en ce que** lesdites première et/ou deuxième et/ou troisième et/ou quatrième séquences d'ADN ont été modifiées en insérant ou en délétant ou en substituant un ou plusieurs nucléotides tout en retenant ou en améliorant l'activité biologique de la séquence d'ADN.

15. Cellule de levure selon l'une quelconque des revendications 1 à 14, **caractérisée en ce que** ladite cassette d'expression est portée par un vecteur circulaire ou linéaire, qui comprend facultativement un ou plusieurs gènes de marqueur de sélection.

16. Cellule de levure selon la revendication 15, **caractérisée en ce que** ledit vecteur comprend l'un quelconque des gènes de marqueur de sélection suivants :
TRP5, LEU2, ADE1, ADE2, HIS3, HIS4, URA3, LYS2, obtenus de préférence à partir de *Saccharomyces, Hansenula, Pichia* ou *Schwanniomyces,* AMY1 ou GAM1, obtenus de préférence à partir de *Schwanniomyces.*

17. Cellule de levure selon l'une quelconque des revendications 15 à 16, **caractérisée en ce que** ledit vecteur comprend de plus une séquence d'ADN capable de contrôler la réplication autonome et le maintien stable du vecteur dans l'organisme hôte.

18. Cellule de levure selon la revendication 17, **caractérisée en ce que** ladite séquence d'ADN est obtenue à partir du génome d'un élément des genres *Saccharomyces, Pichia, Hansenula* ou *Kluyveromyces,* de préférence *Schwanniomyces.*

19. Cellule de levure selon l'une quelconque des revendications 17 et 18, **caractérisée en ce que** ladite séquence d'ADN est choisie parmi les séquences se répliquant de façon autonome ARS, de préférence une ARS obtenue à partir de l'organisme hôte respectif.

20. Cellule de levure selon la revendication 19, **caractérisée en ce que** ladite séquence d'ADN est SwARS1 ou SwARS2, capable de contrôler la réplication autonome et le maintien stable du vecteur dans *Schwanniomyces,* dans laquelle SwARS1 est constituée d'un fragment *Cla*I*Bam*HI adjacent au fragment *Eco*RI présenté à la figure 2a, et SwARS2 est constituée d'un fragment *Eco*RI*-Bgl*II contenu dans le fragment *EcoRI* présenté à la figure 2a.

21. Cellule de levure selon l'une quelconque des revendications 17 à 20, **caractérisée en ce que** ladite séquence d'ADN a été modifiée en délétant, insérant ou substituant un ou plusieurs nucléotides, tout en retenant ou en améliorant sa fonction biologique.

22. Cellule de levure selon l'une quelconque des revendications 1 à 21, **caractérisée en ce que** ladite cassette d'expression est intégrée dans le génome, facultativement en plusieurs copies, de préférence par recombinaison homologue.

23. Procédé pour la préparation d'un polypeptide, dans lequel un organisme hôte de levure est cultivé dans des conditions appropriées et le polypeptide est récupéré d'une manière connue d'elle-même, **caractérisé en ce que** ledit organisme hôte de levure est une cellule de levure selon l'une quelconque des revendications 1 à 22.

24. Procédé selon la revendication 23, **caractérisé en ce que** des cellules de levure de l'espèce *Schwanniomyces occidentalis* sont utilisées en tant qu'organisme hôte et sont de préférence cultivées sur de l'amidon, de la dextrine, du maltose et/ou de la biomasse végétale.

25. Procédé selon l'une quelconque des revendications 23 et 24, **caractérisé en ce que** des cellules de levure du genre *Schwanniomyces* sont utilisées pour la production d'une seule protéine de cellule.

26. Procédé selon l'une quelconque des revendications 23 à 25, **caractérisé en ce que** ledit polypeptide est l'amylase ou la glucoamylase.

27. Utilisation d'une cellule de levure selon l'une quelconque des revendications 1 à 22 pour la transformation et l'expression de gènes étrangers.

## Revendications (Revendications pour l'(les) Etat(s) contractant(s) suivant(s): ES)

1. Procédé pour préparer une cellule de levure capable d'exprimer une protéine étrangère, **caractérisé en ce qu'**au moins une cassette d'expression comprenant :
a) une première séquence d'ADN-servant de régulon,
b) facultativement, une deuxième séquence d'ADN codant pour un peptide signal
c) une troisième séquence d'ADN codant pour une protéine étrangère, et
d) facultativement, une quatrième séquence d'ADN servant de terminateur,
est insérée dans une cellule de levure du genre *Schwanniomyces,* les première, deuxième et quatrième séquences d'ADN étant obtenues à partir d'une levure.

2. Procédé selon la revendication 1, **caractérisé en ce que** lesdites première et/ou deuxième et/ou quatrième séquences d'ADN sont obtenues à partir d'un gène codant pour une enzyme amylolytique.

3. Procédé selon la revendication 2, **caractérisé en ce que** ladite enzyme amylolytique est une α-amylase ou la glucoamylase.

4. Procédé selon l'une quelconque des revendications 1 à 3, **caractérisé en ce que** lesdites première et/ou deuxième et/ou quatrième séquences d'ADN sont obtenues à partir d'une levure d'un élément des genres *Debariomyces, Saccharomycopses, Lipomyces, Pichia* ou *Saccharomyces,* de préférence *Schwanniomyces.*

5. Procédé selon la revendication 4, **caractérisé en ce que** ladite levure est *Schwanniomyces occidentalis.*

6. Procédé selon l'une quelconque des revendications 1 à 5, **caractérisé en ce que** ladite première séquence d'ADN est constituée d'un fragment *Bgl*II*-Xho*I de 1,8 kb, si elle est obtenue à partir du gène de la α-amylase de *Schwanniomyces occidentalis, ou* d'un fragment *Bam*HI*-Pvu*II de 1,3 kb si elle est obtenue à partir du gène de la glucoamylase de *Schwanniomyces occidentalis.*

7. Procédé selon la revendication 5, **caractérisé en ce que** ladite première séquence d'ADN est constituée en partie ou en totalité de la séquence d'ADN correspondant aux bases -1 à -540 de la région précédant le gène structural codant pour la α-amylase, ou en partie ou en totalité de la séquence d'ADN correspondant aux bases -1 à -320 de la région précédant le gène structural codant pour la glucoamylase.

8. Procédé selon l'une quelconque des revendications 1 à 5, **caractérisé en ce que** ladite première séquence d'ADN correspond à l'un des régulons régulant l'expression :
a) de ADH1, ADH2, PDC1, GAL1/10, PGK, GAPDH, le régulon étant de préférence obtenu à partir de *Saccharomyces cerevisiae,*
b) de LAC4, le régulon étant de préférence obtenu à partir de *Kluyveromyces lactis,* ou
c) de régulons correspondants, obtenus de préférence à partir de *Schwanniomyces.*

9. Procédé selon l'une quelconque des revendications 1 à 8, **caractérisé en ce que** ladite deuxième séquence d'ADN code pour tout ou partie d'au moins l'un des peptides suivants :

a)  MetArgPheSerThrGluGlyPheThrSerLysValValAlaAlaIle
LeuAlaPheSerArgLeuValSerAla;

b) MetArgPheSerThrGluGlyPheThrSerLysValValAlaAlaIle
LeuAlaPheSerArgLeuValSerAlaGlnProIleIlePheAspMetArg;

c)  MetIlePheLeuLysLeuIleLysSerIleValIleGlyLeuGlyLeu
ValSerAla;

d)  MetIlePheLeuLysLeuIleLysSerIleValIleGlyLeuGlyLeu
ValSerAlaIleGlnAlaAlaProAla;

e)  MetIlePheLeuLysLeuIleLysSerIleValILeGlyLeuGlyLeu
ValSerAlaIleGlnAlaAlaProAlaSerSerIleGlySerSerAlaSerAla

10. Procédé selon la revendication 9, **caractérisé en ce que** la séquence d'ADN codant pour ledit peptide correspond à une partie ou à la totalité d'une des séquences d'ADN suivantes :

a)  ATGAGATTTTCAACTGAAGGATTTACAAGTAAAGTTGTTGCAGCAATT
TTAGCATTCTCAAGATTGGTATCTGCT,

b) ATGAGATTTTCAACTGAAGGATTTACAAGTAAAGTTGTTGCAGCAATTT
TAGCATTCTCAAGATTGGTATCTGCTCAACCGATTATTTTTGACATGAGA;

c) ATGATTTTTCTGAAGCTGATTAAAAGTATAGTAATTGGTTTGGGATTA
GTTAGTGCT;

d)  ATGATTTTTCTGAAGCTGATTAAAAGTATAGTAATTGGTTTGGGATTA
GTTAGTGCTATCCAAGCAGCCCCTGCC;

e) ATGATTTTTCTGAAGCTGATTAAAAGTATAGTAATTGGTTTGGATTA
GTTAGTGCTATCCAAGCAGCCCCTGCCTCTTCGATTGGATCTAGTGCTTCA
GCA.

11. Procédé selon l'une quelconque des revendications 1 à 10, **caractérisé en ce que** ladite troisième séquence d'ADN codant pour une protéine étrangère est une séquences d'ADN naturelle ou synthétique codant pour une cellulase, une interleukine, un facteur de croissance semblable à l'insuline, un interféron, la lymphokine, un facteur de croissance humain, un facteur de croissance des nerfs, l'aprotinine, l'insuline, l'hirudine, les hormones, les facteurs de coagulation du sang, les antigènes de surface ou de coeur de l'hépatite B, les vaccins viraux ou bactériens, ou le facteur stimulant une colonie de granulocytes/macrophages humain.

12. Procédé selon l'une quelconque des revendications 1 à 11, **caractérisé en ce que** ladite quatrième séquence d'ADN provient d'une levure d'un élément des genres *Saccharomyces, Pichia, Hansenula,* de préférence *Schwanniomyces.*

13. Procédé selon la revendication 12, **caractérisé en ce que** ladite quatrième séquence d'ADN correspond à une partie ou à la totalité du terminateur constitué des nucléotides 1537 à 1740 du gène de la α-amylase de *Schwanniomyces,* ou à une partie ou à la totalité du terminateur constitué des nucléotides 2875 à 3320 du gène de la glucoamylase de *Schwanniomyces.*

14. Procédé selon l'une quelconque des revendications 1 à 13, **caractérisé en ce que** lesdites première et/ou deuxième et/ou troisième et/ou quatrième séquences d'ADN ont été modifiées en insérant ou en délétant ou en substituant un ou plusieurs nucléotides tout en retenant ou en améliorant l'activité biologique de la séquence d'ADN.

15. Procédé selon l'une quelconque des revendications 1 à 14, **caractérisé en ce que** ladite cassette d'expression est portée par un vecteur circulaire ou linéaire, qui comprend facultativement un ou plusieurs gènes de marqueur de sélection.

16. Procédé selon la revendication 15, **caractérisé en ce que** ledit vecteur comprend l'un quelconque des gènes de marqueur de sélection suivants :
TRP5, LEU2, ADE1, ADE2, HIS3, HIS4, URA3, LYS2, obtenus de préférence à partir de *Saccharomyces, Hansenula, Pichia* ou *Schwanniomyces,* AMY1 ou GAM1, obtenus de préférence à partir de *Schwanniomyces.*

17. Procédé selon l'une quelconque des revendications 15 à 16, **caractérisé en ce que** ledit vecteur comprend de plus une séquence d'ADN capable de contrôler la réplication autonome et le maintien stable du vecteur dans l'organisme hôte.

18. Procédé selon la revendication 17, **caractérisé en ce que** ladite séquence d'ADN est obtenue à partir du génome d'un élément des genres *Saccharomyces, Pichia, Hansenula ou Kluyveromyces,* de préférence *Schwanniomyces.*

19. Procédé selon l'une quelconque des revendications 17 et 18, **caractérisé en ce que** ladite séquence d'ADN est choisie parmi les séquences se répliquant de façon autonome ARS, de préférence une ARS obtenue à partir de l'organisme hôte respectif.

20. Procédé selon la revendication 19, **caractérisé en ce que** ladite séquence d'ADN est SwARS1 ou SwARS2, capable de contrôler la réplication autonome et le maintien stable du vecteur dans *Schwanniomyces,* dans laquelle SwARS1 est constituée d'un fragment *Cla*I*-Bam*HI adjacent au fragment *Eco*RI présenté à la figure 2a, et SwARS2 est constituée d'un fragment *Eco*RI-*Bgl*II contenu dans le fragment *Eco*RI présenté à la figure 2a.

21. Procédé selon l'une quelconque des revendications 17 à 20, **caractérisé en ce que** ladite séquence d'ADN a été modifiée en délétant, insérant ou substituant un ou plusieurs nucléotides, tout en retenant ou en améliorant sa fonction biologique.

22. Procédé selon l'une quelconque des revendications 1 à 21, **caractérisé en ce que** ladite cassette d'expression est intégrée dans le génome, facultativement en plusieurs copies, de préférence par recombinaison homologue.

23. Procédé pour la préparation d'un polypeptide, dans lequel un organisme hôte de levure est cultivé dans des conditions appropriées et le polypeptide est récupéré d'une manière connue d'elle-même, **caractérisé en ce que** ledit organisme hôte de levure est une cellule de levure selon l'une quelconque des revendications 1 à 22.

24. Procédé selon la revendication 23, **caractérisé en ce que** des cellules de levure de l'espèce *Schwanniomyces occidentalis* sont utilisées en tant qu'organisme hôte et sont de préférence cultivées sur de l'amidon, de la dextrine, du maltose et/ou de la biomasse végétale.

25. Procédé selon l'une quelconque des revendications 23 et 24, **caractérisé en ce que** des cellules de levure du genre *Schwanniomyces* sont utilisées pour la production d'une seule protéine de cellule.

26. Procédé selon l'une quelconque des revendications 23 à 25, **caractérisé en ce que** ledit polypeptide est l'amylase ou la glucoamylase.

27. Utilisation d'une cellule de levure selon l'une quelconque des revendications 1 à 22 pour la transformation et l'expression de gènes étrangers.
